# EUROPEAN PATENT SPECIFICATION

(11) **EP 1 658 865 B1**
(45) Date of publication and mention of the grant of the patent: **13.01.2010**
(21) Application number: 05257174.2
(22) Date of filing: 22.11.2005
(51) Int. Cl.: A61L 15/60, A61L 15/42, A61L 15/26

(54) **Absorbent hydrogel composites**
Absorbierende Hydrogelverbundstoffe
Composites d'hydrogel absorbantes

(30) Priority: 23.11.2004 GB 0425799
(43) Date of publication of application: 24.05.2006
(73) Proprietor: First Water Limited, Marlborough, Wiltshire SN8 2RB (GB)
(72) Inventor: Munro, Hugh Semple, Chipping Campden GL55 6QT (GB); Andrews, Philip, Marlborough Wiltshire SN8 2RB (GB); Sainz García, Susana, Oxfordshire OX12 9XW (GB)
(74) Representative: Brown, David Leslie

(56) References cited:
- WO-A-92/03172
- WO-A-03/077964
- WO-A-03/092756
- WO-A-2004/052415
- GB-A- 2 369 799
- US-A- 5 846 214

## Description

### Field of the Invention

The present invention relates to absorbent (porous) hydrogel/foam composites, and more particularly to sheet hydrogel/hydrophilic foam composites suitable for use in wound and burn dressings and other applications where a relatively high speed of fluid uptake is required. The invention also relates to processes for the manufacture of the novel hydrogel composites, and to uses of the compositions.

The expressions "hydrogel" and "hydrogel composites" used herein are not to be considered as limited to gels which contain water, but extend generally to all hydrophilic gels and gel composites, including those containing organic non-polymeric components in the absence of water.

### Background of the Invention

WO-A-03/077964 and WO-A-04/052415 (First Water Limited) describe certain superabsorbent hydrogel foam compositions. The composition is a flexible plasticised hydrophilic polymeric matrix. A first portion of the polymeric matrix has an internal cellular structure, and a second portion has a relatively continuous internal structure. The first portion is relatively porous and the second portion is relatively non-porous, relative to each other. The second portion of the composition may include apertures providing fluid flow communication through the said second portion between an external surface of the said second portion and the first portion whereby the first portion of the composition can take up external water or other fluid into the cellular structure through the apertures of the said second portion.

The prior art compositions are preferably provided in sheet form, the portions comprising layers of the sheet.

The said first and second portions of the prior art compositions are preferably (although not essentially) of the same polymeric matrix material and integrally formed in a single polymerisation step.

A large amount of research has been conducted into unfoamed, relatively non-porous, hydrogels based on hydrophilic polymers, e.g. for use as skin adhesives for a range of applications in skin-adhesive articles. Such materials exhibit a range of properties which make them suitable for skin adhesives. Representative references include PCT Patent Applications Nos. WO-97/24149, WO-97/34947, WO-00/06214, WO-00/06215, WO-00/07638, WO-00/46319, WO-00/65143 and WO-01/96422.

While the prior art compositions comprising foam and continuous portions represent a significant development in the area of absorbent hydrogels, particularly but not exclusively for use in skin adhesive applications, nevertheless there is a need for stronger materials having the desired porosity, water retention and other characteristics offered by hydrogel technology.

### Brief Description of the Invention

The present invention is based on our surprising finding that relatively strong absorbent hydrogel/hydrophilic foam composites (hereinafter: "absorbent hydrogel composites" or simply "hydrogel composites") can be made in a convenient manner with very acceptable water uptake speeds. The manufacturing process can be batchwise, partially continuous or continuous. The absorbent hydrogel composites are prepared in sheet or layer form. The hydrogels are characterised by a first hydrophilic absorbent part which has an internal cellular (e.g. foam) structure and a second absorbent part which is relatively continuous (i.e. has a relatively non-cellular internal structure). Some or all of the second part can fill some of the voids of the internal cellular structure of the first part. The second part can have apertures provided therethrough, to assist uptake of water and other fluids into the first part. The composites are not formed integrally together in one step from a single pre-gel mixture, but must be assembled. The sheet or layer composites may be supported on a relatively strong polymeric support sheet, which may be permeable or impermeable to moisture liquid or moisture vapour. The absorbent hydrogel composites can combine the requirements of good gel flexibility, good mechanical strength and good fluid absorption capacity, optionally also with tackiness to the skin.

The expressions "comonomer", "monomer" and like expressions used herein include ionic and non-ionic monomers and monomer mixtures. Correspondingly, the expressions "polymerize", "polymers" and like expressions include both homopolymerisation and copolymerisation, and the products thereof.

The expressions "sheet" and "layer" used herein include portions of sheets and layers, respectively, unless specifically stated otherwise.

According to a first aspect of the present invention, there is provided an absorbent material or article comprising an absorbent hydrogel composite sheet including: a first hydrophilic absorbent layer part having two major faces and having an internal cellular structure and a second part which comprises a flexible plasticised hydrophilic polymer matrix having an internal structure which is relatively continuous in relation to the internal structure of the first part, provided that at least one of the said first part and the said second part comprises a hydrogel; wherein the first part and the second part arc not formed integrally together in one step from a single pre-gel mixture and the second part is associated with the layer part such that the second part either:
(a) infiltrates some but not all of the cellular voids of the first hydrophilic absorbent layer part over a first major face of the absorbent layer part; or
(b) overlies a major face of the absorbent layer part whereby some but not all of the first part is effectively exposed to permit absorption of external water or other fluid into the cellular voids through the face of the sheet corresponding to that said major face of the absorbent layer.

In an embodiment of the first aspect, there is provided on absorbent material or article comprising an absorbent hydrogel composite sheet including: a first, absorbent layer, part having two major faces which comprises a flexible plasticised hydrophilic polymer matrix having an internal cellular structure and a second part which comprises a flexible plasticised hydrophilic polymer matrix having an internal structure which is relatively continuous in relation to the internal structure of the first part, provided that at least one of the flexible plasticised hydrophobic polymer matrices of the said first, absorbent layer, part and the said second part comprises a hydrogel; wherein the first, absorbent layer part and the second part arc not formed integrally together in one step from a single pre-gel mixture and the second part is associated with the layer part such that the second part either:
(a) infiltrates some but not all of the cellular voids of the first, absorbent layer, part over a first major face of the absorbent layer part; or
(b) overlies a major face of the absorbent layer part whereby some but not all of the first, absorbent layer, part is effectively exposed to permit absorption of external water or other fluid into the cellular voids through the face of the sheet corresponding to that said major face of the absorbent layer.

The flexible plasticised hydrophilic polymer matrix materials forming the said absorbent layer and second parts of the absorbent hydrogel composite sheet in the embodiment above may be the same or different.

The absorbent layer may suitably comprise a hydrophilic polyurethane foam or a hydrogel.

The second part of the absorbent hydrogel composite sheet may suitably comprise a hydrogel and infiltrates some but not all of the cellular voids of the absorbent layer part. To achieve this in manufacture, typically the cellular voids are typically established in the solid matrix forming the absorbent layer part of the composite before ingress of a liquid pre-gel material for forming the second part of the composite. That portion of the said second part of the composite which infiltrates some but not all of the cellular voids of the absorbent layer part of the composite may be all or only some of the said second part of the composite. When it is only some of the said second part of the composite, it may preferably be continuous with the remainder of the said second part of the composite, i.e. that portion which does not infiltrate the cellular voids of the absorbent layer part of the composite. The weight of the second part of the absorbent hydrogel composite per unit area ("coat weight") is typically in the range of up to 500 g/m², for example 50 to 460 g/m², 150 to 460 g/m², or 200 g/m².

The second part of the composite is preferably absorbent, and may in particular have a water absorption capacity generally similar to the absorbent layer part of the composite having the internal cellular structure.

In accordance with the first aspect of the present invention, some but not all of the absorbent layer part of the hydrogel composite is effectively exposed for absorption of external water or other fluid through one side of the sheet. In addition, the absorbent layer part of the hydrogel composite may be effectively exposed for absorption of external water or other fluid through the other side of the sheet, or may be effectively sealed from absorption of external water or other fluid through the other side of the sheet, or some regions of the absorbent layer part may be effectively exposed and other regions of the absorbent layer part may be effectively sealed with respect to absorption of external water or other fluid through the other side of the sheet. This may be achieved by overlaying the said other side of the sheet with a support layer of a polymeric material which may be, respectively, permeable to moisture liquid and/or moisture vapour, impermeable to moisture liquid and/or moisture vapour, or may have regions which are permeable to moisture liquid and/or moisture vapour and regions which are impermeable to moisture liquid and/or moisture vapour. The said support layer, when present, is preferably a mechanically strong polymeric supporting sheet or film. This preferably comprises a non-hydrogel polymer. The said support layer may suitably comprise a backing layer of a sheet absorbent article such as a wound or burn dressing. Where such a support layer is present, one or more additional layer (e.g. of relatively continuous hydrogel material, for example of the same general type as the material forming the said second part of the hydrogel composite sheet, which may if desired infiltrate some but not all of the cellular voids of the absorbent layer part) may if desired be present, between the absorbent layer part and the support layer.

The use of a support layer of a polymeric material is itself novel in a more general sense in connection with foam/continuous composites of the type with which the present invention is concerned.

Therefore, in one embodiment, there is provided an absorbent material or article comprising an absorbent hydrogel composite sheet including: an absorbent layer part having two major faces, which comprises a flexible plasticised hydrophilic polymer matrix having an internal cellular structure, and a second part which comprises a flexible plasticised hydrophilic polymer matrix having a relatively continuous internal structure; wherein the second part is associated with the layer part such that the second part overlies a major face of the absorbent layer part such that some but not all of the absorbent layer part is effectively exposed for absorption of external water or other fluid through the side of the sheet corresponding to that major face of the absorbent layer part which is overlain by the second part, and wherein there is provided over the other major face of the layer part a support layer of a polymeric material.

In this embodiment and when the second part of the hydrogel composite substantially entirely overlies the said major face of the layer part and some but not all of the absorbent layer part is effectively exposed for absorption of external water or other fluid by virtue of apertures provided through the second part which provide fluid flow communication through the second part between an external surface of the second part and the absorbent layer part, whereby the layer part can take up external water or other fluid into the cellular structure through the apertures of the second part, then the apertures of the second part may continue through the absorbent layer part to penetrate it entirely, but without penetrating the support layer provided over the other major face of the absorbent layer part.

In the embodiment of the present invention, generally speaking parts corresponding to the parts present in the first aspect of the invention may be provided in the same way and with the same embodiments, examples and preferences. Thus, for example, the said second part of the hydrogel composite may infiltrate some but not all of the cellular voids of the absorbent layer part, or the said second part of the hydrogel composite may be associated with the absorbent layer part in such a way - e.g. lamination, with or without intermediate adhesives or other components - that it does not infiltrate any of the cellular voids of the absorbent layer part

Further, in the embodiment of the present invention, the support layer may, for example, be permeable to moisture liquid and/or moisture vapour, impermeable to moisture liquid and/or moisture vapour, or may have regions which are permeable to moisture liquid and/or moisture vapour and regions which are impermeable to moisture liquid and/or moisture vapour. The said support layer, when present, is preferably a mechanically strong polymeric supporting sheet or film. This preferably comprises a non-hydrogel polymer. The said support layer may suitably comprise a backing layer of a sheet absorbent article such as a wound or burn dressing. Where such a support layer is present, one or more additional layer (e.g. of relatively continuous hydrogel material, for example of the same general type as the material forming the said second part of the hydrogel composite sheet, which may if desired infiltrate some but not all of the cellular voids of the absorbent layer part) may if desired be present, between the absorbent layer part and the support layer.

The support layer is formed of any suitable material, e.g. a polymer (which may be foamed or unfoamed, or any combination thereof) such as polyurethane, or a fabric (which may comprise natural fibres, synthetic fibres or any combination thereof, and may be woven or non-woven). The support layer may have any suitable structure, e.g. a web, film, sheet, net or any combination thereof.

According to the invention, the second part of the hydrogel composite sheet overlies a major face of the absorbent layer part such that some but not all of the absorbent layer part is effectively exposed for absorption of external water or other fluid through the side of the sheet corresponding to that major face of the absorbent layer part which is overlain by the second part. The expression "effectively exposed" and like expressions, used herein, means that external water or other fluid at the said side of the sheet can gain access into the cellular internal structure of the absorbent layer part of the hydrogel composite sheet for absorption into the composite sheet within an acceptable period of time and in acceptable volumes according to the intended use of the composite sheet. It is not essential that the external water or other fluid must physically contact the absorbent layer part of the hydrogel composite sheet before any other part; for example, a thin layer of a hydrogel having a continuous internal structure or other absorbent material may be disposed over an "effectively exposed" portion of the absorbent layer of the hydrogel composite - provided that the external water or other fluid can pass through any such thin extra layer within an acceptable period of time and in acceptable volumes according to the intended use of the composite, and thereby into the cellular internal structure of the composite sheet, then the requirements of the invention would have been fulfilled. It is also not necessary that an "effectively exposed" portion of the absorbent layer part is outward facing to the side of the composite sheet through which the external water or other fluid can be absorbed - by means of three-dimensional surface and/or internal features provided on the composite sheet (such as, for example, apertures, hollows, dents, slits, slots, depressions, raised bumps, raised ridges, or any combination thereof), portions of the absorbent layer part of the hydrogel composite sheet can be effectively exposed in such a way that external water or other fluid can pass into the cellular internal structure within an acceptable period of time and in acceptable volumes according to the intended use of the composite, for example passing around surface regions of the hydrogel material constituting the second part of the composite. Such three-dimensional features may advantageously invade the absorbent layer part of the composite to some extent to achieve their exposing effect. They may even penetrate the absorbent layer part of the hydrogel composite sheet entirely, particularly (as mentioned above) if a support layer is provided over the opposite major face of the composite sheet.

The absorbent layer part of the hydrogel composite sheet may comprise a porous foam having an internal cellular structure such that the volume ratio of cell void to matrix is greater than about 1:3, more preferably greater than about 1:1, and the second part may comprise a relatively continuous matrix, which may have substantially no cell voids or only occasional cell voids (e.g. a volume ratio of cell void to matrix less than about 1:10, for example less than about 1:20). The said second part of the hydrogel composition will be referred to herein as "continuous", which expression is used in the relative sense explained above.

It is preferred that the absorbent layer part of the composite has a first water uptake rate and the second part of the composite has a second water uptake rate (disregarding any apertures or the like providing fluid flow communication). The first and second water uptake rates may be generally similar. At least the first water uptake rate may be very fast, e.g. comparable with the rate of absorption of water by absorbent paper kitchen roll. The absorption capacity of the hydrogel composite will generally be at least about 30% by weight (i.e. the weight of water taken up and held at saturation will be at least about 30% of the weight of the hydrogel composite used), and may be as much as about 20000%. More typically, the absorption capacity of the hydrogel composite will be between about 300% and about 10000%.

A skin-tacky hydrogel composite sheet according to the present invention may be used in a bioadhesive article which is adapted to be adhered to human or other mammalian skin in use. Such an article typically comprises the hydrogel composite sheet as an adhesive for contacting the skin and a substrate supporting the hydrogel adhesive sheet. The side of the hydrogel composite sheet through which external water or other fluid can be absorbed into the sheet typically defines the skin-contactable surface of the hydrogel composite sheet. The skin-contactable surface of the hydrogel composite is protected prior to use by an overlying release layer. Examples of articles in which the hydrogel composite sheet according to the present invention may be used are set out in the Detailed Description of the Invention.

It is found that the absorbent material of the present invention absorbs moisture well into the cellular structure, while loss of moisture from the material can be restricted or controlled so that the material serves in use also as a moisture reservoir, which is beneficial to human or other mammalian skin in a range of circumstances, as discussed in more detail below. Moreover, the infiltration of voids of the cellular structure by the material of the relatively non-absorbent second part of the composite strengthens and reinforces the composite material, this strengthening being assisted by any support layer present.

According to a second aspect of the present invention, there is provided a process for the preparation of an absorbent hydrogel composite sheet comprising associating a first hydrophilic absorbent layer, part having two major faces and having an internal cellular structure and second part which comprises a flexible plasticised hydrophilic polymer matrix having an internal structure which is relatively continuous in relation to the internal structure of the first part, provided that at least one of the said first part and the said second part comprises a hydrogel such that the second part either:
(a) infiltrates some but not all of the cellular voids of the first hydrophilic absorbent layer part over a first major face of the absorbent layer part; and/or b) overlies a major face of the absorbent layer part whereby some but not all of the first part is effectively exposed to permit absorption of external water or other fluid into the cellular voids through the face of the sheet corresponding to that said major face of the absorbent layer, and wherein the first, part and the second part are not formed integrally together in one step from a single pre-gel mixture.

In an embodiment of the first aspect, there is provided a process for the preparation of an absorbent hydrogel composite sheet, comprising associating a first, absorbent layer, part having two major faces which comprises a flexible plasticised hydrophilic polymer matrix having an internal cellular structure and second part which comprises a flexible plasticised hydrophilic polymer matrix having an internal structure which is relatively continuous in relation to the internal structure of the first part, provided that at least one of the flexible plasticised hydrophobic polymer matrices of the said first, absorbent layer, part and the said second part comprises a hydrogel such that the second part either:
(a) infiltrates some but not all of the cellular voids of the first, absorbent layer, part over a first major face of the absorbent layer part; and/or
b) overlies a major face of the absorbent layer part whereby some but not all of the first, absorbent layer, part is effectively exposed to permit absorption of external water or other fluid into the cellular voids through the face of the sheet corresponding to that said major face of the absorbent layer, and wherein the first, absorbent layer part and the second part are not formed integrally together in one step from a single pre-gel mixture.

Where the said second part of the composite sheet is to be infiltrated into the absorbent layer part, association of the parts is preferably achieved by first forming the absorbent layer part of the composite as a foam layer in the solid cured state, and subsequently applying to a surface thereof a liquid pre-gel precursor for the second part of the composite, allowing the liquid pre-gel precursor to infiltrate the pores of the absorbent layer part to a desired extent, and subsequently curing the second part to provide the composite sheet.

Where the said second part of the composite sheet is not to be infiltrated into the absorbent layer part, association of the parts is preferably achieved by adhering or laminating in conventional manner pre-formed areas of the second part of the composite to a pre-formed absorbent layer part of the composite (e.g. a foam layer in the solid cured state).

Other layers or areas - e.g. one or more additional hydrogel layer or area - may if desired be provided on one or both sides of the hydrogel composite sheet according to the present invention, or may be interposed between the parts of the hydrogel composite sheet according to the present invention. Such other layers or areas may be foamed or unfoamed, hydrogel or non-hydrogel. An additional layer of an unfoamed (continuous) hydrogel may, for example, be provided overlying the absorbent layer part of the hydrogel composite sheet, and particularly between the absorbent layer part of the hydrogel composite sheet and any support layer present. The hydrogel of such an additional layer, when present, may conveniently be selected from the same hydrogel materials as those from which the second part of the hydrogel composite sheet may be formed, as described herein, or from other unfoamed hydrogels as described in any of the prior art documents acknowledged herein. Such additional layer(s), when present, may be associated with the essential components of the hydrogel composite sheet in any suitable manner. The means of association include infiltration where appropriate, lamination where appropriate, or in some cases an absorbent bilayer or multi-layer component can be prepared in one procedure as an integral unitary material, using the methods described in WO-A-03/77964 and WO-A-2004/052415.

A scrim material may be present, suitably within any one or more relatively continuous hydrogel layers that are present in the material or article according to the present invention. Such a scrim material may be formed of a material that is natural in origin, synthetic in origin, or partly natural and partly synthetic. The scrim may suitably be in the form of a net or a woven or non-woven fabric. Preferred scrims include those formed from polyolefins, polyamides, polyacrylates, or polyesters, for example non-wovens or nets. The crim material may, for example, comprise sodium polyacrylate fibres, such as those commercially available under the tradename Oasis™ from Acordis Technical Absorbents Limited. The scrim is preferably provided by introducing it into a laid down (e.g. cast) layer of a pre-gel liquid precursor for the hydrogel layer, before curing, so that the liquid pre-gel covers and surrounds the scrim. On curing of the liquid pre-gel, the hydrogel is thereby formed encapsulating the scrim material. Use of a scrim material in this way is found to be potentially helpful in enhancing the strength and ease of handling of the hydrogel component and/or the finished composite.

The absorbent hydrogel composite sheet of the material or article of the first aspect of the present invention may preferably consist essentially of:
(a) the absorbent layer part, the second part disposed on some but not all of one major face of the absorbent layer (optionally with three-dimensional surface features, e.g. selected from depressions, slits, crosses or any combination thereof provided on said major face), and a support layer adhered to the other major face of the absorbent layer; or
(b) as (a) and an additional continuous hydrogel layer disposed between the absorbent layer and the support layer; or
(c) the absorbent layer part, the second part disposed on all of one major face of the absorbent layer, and a support layer adhered to the other major face of the absorbent layer, with apertures penetrating the second part and the full thickness of the absorbent layer up to, but not including, the support layer; or
(d) as (c) and an additional continuous hydrogel layer disposed between the absorbent layer and the support layer, the apertures penetrating also the additional continuous hydrogel layer; or
(e) the absorbent layer part, and the second part disposed in depressions provided in one major face of the absorbent layer part so that the second part covers some but not all of that major face of the absorbent layer, and a support layer adhered to the other major face of the absorbent layer; or
(f) as (e) and an additional continuous hydrogel layer disposed between the absorbent layer and the support layer; or
(g) the absorbent layer part, the second part printed on some but not all of one major face of the absorbent layer, and a support layer adhered to the other major face of the absorbent layer; or
(h) as (g) and an additional continuous hydrogel layer disposed between the absorbent layer and the support layer; or
(i) as any one of (a) to (h) and a scrim material disposed within the second part; or
(j) as any one of (b), (d), (f), (h) and (i) (to the extent that (i) refers back to any of (b), (d), (f) and (h)) and a scrim material disposed within the additional continuous hydrogel layer.

The hydrogel components of the embodiments (a) to (j) above are associated together by any means within the options provided for in the first and second embodiments of the present invention defined above.

For forming any required component part of the composite sheet, generally conventional preparation methods, or methods described in the prior art acknowledged above, may be used. Curing of a pre-gel can suitably be by polymerisation, although evaporation of a solvent from a liquid pre-gel which comprises an organic solvent solution of a pre-formed polymer - to leave behind a dry residue of the polymer - is not excluded from the ambit of the word "curing" as used herein. Thus, for example, the absorbent layer part of the hydrogel composite for use in the present invention may thus generally be prepared by a process which comprises polymerising a polymerisable mixture comprising a hydrophilic monomer selected from monomers and monomer mixtures, wherein the polymerisable mixture includes introduced gas bubbles, as described in WO-A-03/077964 and WO-A-2004/052415.

Any polymerisation step in the manufacture of the composite hydrogel sheet or component parts thereof is preferably a free radical polymerisation performed in air using a polymerisation inducing device such as a heat, light (e.g. UV light) or other radiation source which is in relative motion with respect to the polymerisable mixture. In this way, a moving line-wise polymerisation procedure can take place, rather than the static batchwise procedures available from the prior art. The polymerisable mixture or composite of pre-formed part (e.g. cured foamed component) and polymerisable pre-gel (herein generically: "polymerisable material") is laid down in sheet or layer form on a suitable support arrangement for the polymerisation procedure.

Most preferably, the support arrangement comprises a structure which underlies and supports a sheet material adapted to receive the laid down polymerisable material, the sheet material being removable from the underlying structure, e.g. after completion of polymerisation, and the said projections extending from the upper surface of the sheet material. The sheet material may have a non-stick surface, so that it may easily be removed from the hydrogel composition after completion of polymerisation. The sheet material is preferably adapted to constitute a release layer in conventional manner, for protecting the polymerised hydrogel composite sheet prior to use.

A similar support arrangement may also conveniently be used, in the case where the hydrogel composite is prepared by adhesion or lamination of pre-formed hydrogel parts together.

The resultant hydrogel composite and the release layer may be maintained in contact with each other in a subsequent process for manufacturing an article including the hydrogel composite.

Alternatively, a further release layer may suitably be applied to the exposed surface of the freshly prepared hydrogel composite, to protect the same for storage or transportation. At the times of subsequent processing and use, the relevant release layer is peeled away and may be discarded.

Any required three-dimensional surface and/or internal features of the hydrogel composite or any part thereof may suitably be formed simultaneously with, or after, formation of the polymer matrix of the said part or simultaneously with, or after, formation of the composite sheet itself. Such features may, for example, be formed by moulding, punching, cutting, slicing and/or pressing, in conventional manner. Component parts of the composite can be associated with one another in conventional manner (e.g. using adhesives or other conventional bonding methods), or parts (e.g. layers) can be formed *in situ* on other component parts. For example, apertures and similar features can be formed *in situ* during any polymerisation reaction using an appropriately shaped underlying support sheet of the support arrangement for the pre-gel material, as described in more detail in WO-A- 2004/052415.

The absorbent hydrogel composites used in this invention may be electrically conductive and constitute a skin-contactable, preferably adhesive, portion of a biomedical electrode. Such a hydrogel composition will typically provide good current dispersion over the skin-electrode interface.

According to a further aspect of the present invention, there is provided a biomedical electrode comprising an electrically conductive current distribution member adapted for electrical connection to an electrical apparatus, and an electrically conductive skin contactable portion in association with the electrically conductive current distribution member, whereby electrical current can flow between the electrical apparatus and a wearer's skin when the electrode is in use, wherein the electrically conductive skin contactable portion comprises an absorbent hydrogel composite or a hydrogel/release layer combination according to the first aspect of the present invention or prepared according to the second aspect of the present invention. The electrically conductive skin contactable portion is in sheet or layer form. The side of the composite through which external water or other fluid can be absorbed will preferably form the skin-contactable surface of the electrically conductive skin contactable portion.

A bioadhesive wound or burn dressing typically comprises an absorbent member adapted to contact a wearer's skin in the location of a wound or burn, and a sheet backing member supporting the absorbent member, the sheet backing member including a portion which extends beyond the absorbent member and defines a skin-directed surface which carries a pressure-sensitive adhesive for securement of the dressing to the wearer's skin.

According to a further aspect of the present invention, there is provided a wound or burn dressing which comprises an absorbent member adapted to contact a wearer's skin in the location of a wound or burn, and a sheet backing member supporting the absorbent member, the sheet backing member including a portion which extends beyond the absorbent member and defines a skin-directed surface which carries a pressure-sensitive adhesive for securement of the dressing to the wearer's skin, wherein the absorbent member comprises an absorbent hydrogel composite or a hydrogel/release layer combination according to the first aspect of the present invention or prepared according to the second aspect of the present invention. The absorbent member is in sheet or layer form. The side of the composite through which external water or other fluid can be absorbed will preferably form the skin-contactable surface of the absorbent member.

The sheet backing member is formed of any suitable material, e.g. a polymer (which may be foamed or unfoamed, or any combination thereof) such as polyurethane, or a fabric (which may comprise natural fibres, synthetic fibres or any combination thereof, and may be woven or non-woven). The sheet backing member may have any suitable structure, e.g. a web, film, sheet, net or any combination thereof. The sheet backing member in this wound and burn dressing aspect of the invention is preferably the support member part of the absorbent material of the first aspect of the present invention, simply dimensioned so as to extend beyond the absorbent member which comprises the absorbent hydrogel composite sheet according to the invention.

The pressure-sensitive adhesive is any suitable skin-compatible adhesive, e.g. a known acrylic-based polymeric pressure-sensitive adhesive; or a bioadhesive hydrogel or gel or a bioadhesive porous plasticised hydrophilic polymer having an internal cellular structure, such as those described in the PCT Patent Applications mentioned above.

### Detailed Description of the Invention

### Internal Structure of the Hydrogel Parts

The internal cellular structure of the absorbent layer part may be closed-cell throughout, open-cell throughout, or may have regions of closed-cell structure and regions of open-cell structure. Generally speaking, an open-cell structure is preferred, as it will absorb fluid at a faster initial rate than a closed-cell structure, by reason of the interconnection of the internal cells.

The second part or any additional continuous layer of the hydrogel composite sheet may suitably be in the form of a layer of the sheet, which may be of the same or different material to the absorbent layer. The layers may be infiltrated together, as described above, or may be laminated together, optionally with intermediate bonding media.

### Construction of the Composite Sheet

The absorbent layer part is preferably pre-formed as described in the prior art mentioned above.

Where it is desired that the absorbent hydrogel composite sheet consists essentially of the absorbent layer and the infiltrated relatively non-absorbent second part, it is preferred that the absorbent layer is formed in such a way that substantially no "draining" of the foamed (gassed) pre-gel takes place, as described in WO-A-03/077964 and WO-A-2004/052415, so that the formation of a relatively continuous (non-absorbent) portion adjacent the foamed layer is minimised. The infiltrated relatively non-absorbent part can then be formed on the absorbent layer as described in more detail below.

Where it is desired that the absorbent hydrogel composite sheet comprises the absorbent layer, the infiltrated relatively non-absorbent part, and a further relatively non-absorbent part (e.g. layer) overlying the absorbent layer, the absorbent layer may be formed in such a way that "draining" of the foamed (gassed) pre-gel takes place, so that a relatively continuous (non-absorbent) portion adjacent the foamed layer is simultaneously formed, which can the serve as the further relatively non-absorbent layer overlying the absorbent layer. The infiltrated relatively non-absorbent part would be formed on the absorbent portion of the resulting material as described in more detail below.

Where the components of the hydrogel composite sheet are associated together by lamination without substantial infiltration of one hydrogel material into voids of another, the lamination can be accomplished in generally conventional manner, typically by using the typical inherent tackiness of plasticised hydrogels or by using an intermediate bonding adhesive between the parts (e.g. a conventional acrylic adhesive), or a combination of both.

The hydrogel composite sheet preferably includes a non-hydrogel support layer (e.g. of polyurethane or the like) associated with the side opposite to the side from which water can be absorbed (the latter side being typically the skin-contacting side). The composite is preferably protected before use by the skin-contacting side being in contact with a protective release layer, for example siliconised plastic or paper. Alternatively, the hydrogel composite may be provided, for example, in partially finished form in the form of a sheet having first and second major hydrogel faces, i.e. with no support layer, both of said hydrogel faces being in contact with a protective release layer, for example siliconised plastic or paper.

Where the hydrogel composite is present in an article, the support layer is preferably a backing member for the article, e.g. a backing member forming part of a wound or burn dressing, a biomedical electrode or another article. Particularly preferred are articles where a bioadhesive hydrogel layer is to be provided in use between the article and the skin of a patient. Such articles are exemplified below (see "Applications"). Still further, as previously mentioned, the hydrogel composition may be present in the form of a sheet having a woven or non-woven fabric, or a net, embedded therein.

The hydrogel sheets may typically have a substantially uniform thickness. The hydrogel sheets may typically have a thickness in the range of about 0.5 mm to about 10 mm. The hydrogel composition may suitably be in the form of a sheet having a mean basis weight of hydrogel in the range of about 0.1 kg/m² to about 2.5 kg/m².

When the hydrogel composition contains water, the water may be present in any suitable amount. The typical range of water content is between 0 and about 95% by weight of the hydrogel. The hydrogel composition may conveniently be classified as "high water content" or "low water content". The expression "high water content" refers particularly to hydrogel compositions comprising more than about 40% by weight of water, more particularly above about 50% by weight, and most preferably between about 60% and about 95% by weight. The expression "low water content" refers particularly to hydrogel compositions comprising up to about 40% by weight of water.

Any three-dimensional surface features of the hydrogel composite are preferably formed by moulding at the stage of preparation of the composite sheet, or alternatively by stamping, punching and/or cutting a pre-assembled flat composite sheet, using a conventional stamping, punching and/or cutting apparatus. In this second alternative method, the flat composite sheet is preferably formed initially with a uniform layer of the hydrogel for providing the second part of the composite disposed over the full area of the face of the absorbent layer through which it is desired that external water or other fluid is to be capable of being absorbed. The process of stamping, punching and/or cutting the flat composite sheet to form the three-dimensional surface features typically breaks the uniform layer of the hydrogel and thereby creates the effective exposure of the underlying absorbent layer, in accordance with the invention. Other ways of constructing the composite structures of the present invention will be readily apparent to those of ordinary skill in this art, and need not be discussed in detail here; generally speaking, the methods for preparing the composite material and articles of the present invention typically use liquid pre-gel deposition methods such as casting, injecting, drop application or printing (e.g. screen printing or gravure printing) and subsequent curing, in combination with conventional liquid shaping methods such as moulding, and/or mechanical forming of solid pre-formed sheet components (e.g. punching, stamping and/or cutting), to form single layers, a partially assembled layer structure or the completely assembled layer structure, and if necessary subsequently associating component parts together and/or forming parts or layers from liquid pre-gel materials by similar methods *in situ* on pre-formed component parts and subsequent curing.

The surface features of the hydrogel sheet are preferably provided in a grid or array across the surface of the hydrogel composite. In the case of a composite for use in a wound or burn dressing, any apertures may preferably be spaced far enough apart from each other to effectively restrict granulation (scab formation) between adjacent apertures when in contact with a wound. Typically, the surface features (e.g. depressions, apertures, etc.) may be between about 0.5 and about 1.5 cm apart, more preferably between about 0.6 and about 1.0 cm apart. The surface features are preferably tapered so that their external ends are somewhat (e.g. between about 20% and about 1000%) wider than their internal ends. In this way, allowing for the inherent flexibility of the polymeric matrix material, the surface features can preferentially permit fluid flow from the wearer's skin to the absorbent layer of the hydrogel composite, in comparison to the reverse direction. Fluid flow in the reverse direction would tend to close the internal end of the aperture, obstructing the flow. Such a one-way effect assists in preventing leakage of fluid from the absorbent layer of the hydrogel, when in use it functions as a fluid reservoir.

### The Hydrogel Composite - Physical Parameters

The density of the hydrogel composites of the present invention can be selected within a wide range, according to the materials used and the manufacturing conditions. Generally speaking, the bulk density of the total hydrogel composite may be in the range of about 0.05 to about 1.5g/cm³, more typically in the range of about 0.3 to about 0.8g/cm³.

The water activity of the hydrogel composites of the present invention typically lies within the range of 0 to about 0.96, as measured by an AquaLab Series 3TE water activity meter.

The water uptake rate of the absorbent layer and the second or any additional continuous parts of the hydrogel composites of the present invention typically lies within the range of at least about 2 µl/s, more preferably between about 2 and about 100µl/s, as measured by the technique of applying a 5µl drop of water from a syringe onto the face of the sheet hydrogel and measuring the reduction in volume of the drop over a period of 0.1s starting from contact between the drop and the hydrogel, and extrapolating to a rate expressed as volume per second, the measurements being made using a Scientific and Medical Products DAT1100 dynamic contact angle analyser. A water uptake rate of, say, 25µl/s, indicates complete absorption of the applied water in 0.2s.

The absorption capacity of the hydrogel composite will generally be between about 30% and about 20000%. More typically, the absorption capacity of the hydrogel composite will be between about 300% and about 10000%.

### Ingredients of the Hydrogel Composition

The preferred hydrogel composition of the present invention comprises a plasticised three-dimensional matrix of cross-linked polymer molecules, and has sufficient structural integrity to be self-supporting even at very high levels of internal water content, with sufficient flexibility to conform to the surface contours of the human skin. Where the intended use of the hydrogel is in biomedical electrodes, wound dressings, and other applications where skin adhesion is desired, the hydrogel composition preferably has sufficient bioadhesion to adhere to the skin under all skin and moisture conditions likely to be encountered during use. Our PCT Patent Application No. WO-00/45864, describes a method whereby the skin adhesion performance of the hydrogel can be predicted and thereby tailored to particular applications.

The hydrogel composites with which the present invention is concerned generally comprise, in addition to the cross-linked polymeric network, an aqueous plasticising medium and, where electrical conductivity is required, at least one electrolyte. The materials and processing methods used are normally chosen to provide a suitable balance of adhesive and electrical properties for the desired application. For further details of the materials and methods of manufacture of individual component parts, please refer to the prior art documents acknowledged herein, as well as standard texts on hydrogels (e.g. "Hydrogels" in Kirk-Othmer Encyclopedia of Chemical Technology, 4th Edition, vol. 7, pp. 783-907, John Wiley and Sons, New York.

### Monomers

The hydrogel component material may, for example, be a polymer of one or more ionic and/or non-ionic monomer.

A suitable ionic monomer will be water soluble and may be selected from: 2-acrylamido-2-methylpropane sulphonic acid or an analogue thereof or one of its salts (e.g. an ammonium or alkali metal salt such as a sodium, potassium or lithium salts); acrylic acid or an analogue thereof or one of its salts (e.g. an alkali metal salt such as a sodium, potassium or lithium salt); and/or a polymerisable sulphonate or a salt thereof (e.g. an alkali metal salt such as a sodium, potassium or lithium salt), more particularly acrylic acid (3-sulphopropyl) ester or an analogue thereof, or a salt thereof. The term "analogue" in this context refers particularly to substituted derivatives of 2-acrylamido-2-methylpropane sulphonic acid, of acrylic acid or of acrylic acid (3-sulphopropyl) ester, as described in more detail in the acknowledged prior art.

A further category of ionic monomer that may be mentioned is a monomer/comonomer pair consisting of a first monomer comprising one or more pendant anionic group and a second monomer comprising one or more pendant cationic group, the relative amounts of the said monomers in the pair being such that the anionic groups and cationic groups are present in essentially equimolar quantities. The said anionic and cationic groups may be selected from groups which are salts of acid groups and groups which are salts of basic groups. The pendant groups in the first monomer are preferably the sodium, potassium, calcium, lithium and/or ammonium (individually or in any combination of one or more) salts of carboxylic acid, phosphoric acid and/or sulphonic acid. Sulphonic acid groups are most preferred. The pendant groups in the second monomer are preferably quaternary ammonium salts of halide (for example chloride), sulphate and/or hydroxide. Chloride and sulphate are most preferred.

A particularly preferred ionic monomer is a sodium salt of 2-acrylamido-2-methylpropane sulphonic acid, commonly known as NaAMPS, which is available commercially at present from Lubrizol as either a 50% aqueous solution (reference code LZ2405) or a 58% aqueous solution (reference code LZ2405A) and/or acrylic acid (3-sulphopropyl) ester potassium salt, commonly known as SPA or SPAK. SPA or SPAK is available commercially in the form of a pure solid from Raschig.

A suitable non-ionic monomer, if present, may preferably be water soluble and be selected from acrylamide or a mono- or di-N-alkylacrylamide or an analogue thereof. The term "analogue" in this in this context refers to non-ionic water soluble monomers containing an alkyl or substituted alkyl group linked to a carbon-carbon double bond via an amido or alkylamido (-CO.NH- or -CO.NR-) function. Examples of such analogues include diacetone acrylamide (N-1,1-dimethyl-3-oxobutyl-acrylamide), vinyl lactams, N-alkylated acrylamides, N,N-dialkylated acrylamides, N-vinyl pyrrolidone, N-acryloyl morpholine and any mixture thereof (particularly N-acryloyl morpholine).

Optional substituents of the monomers used to prepare the hydrogels used in the present invention may preferably be selected from substituents which are known in the art or are reasonably expected to provide polymerisable monomers which form hydrogel polymers having the properties necessary for the present invention. Suitable substituents include, for example, lower (C1 to C6) alkyl, hydroxy, halo and amino groups.

### Cross-linking Agents

Conventional cross-linking agents are suitably used to provide the necessary mechanical stability and to control the adhesive properties of the hydrogel. The amount of cross-linking agent required will be readily apparent to those skilled in the art such as from about 0.01 % to about 0.5%, particularly from about 0.05% to about 0.4%, most particularly from about 0.08% to about 0.3%, by weight of the total polymerisation reaction mixture. Typical cross-linkers include tripropylene glycol diacrylate, ethylene glycol dimethacrylate, triacrylate, polyethylene glycol diacrylate (polyethylene glycol (PEG) molecular weight between about 100 and about 4000, for example PEG400 or PEG600), and methylene bis acrylamide.

### Organic Plasticisers

The one or more organic plasticiser, when present, may suitably comprise any of the following either alone or in combination: at least one polyhydric alcohol (such as glycerol, polyethylene glycol, or sorbitol), at least one ester derived therefrom, at least one polymeric alcohol (such as polyethylene oxide) and/or at least one mono- or poly-alkylated derivative of a polyhydric or polymeric alcohol (such as alkylated polyethylene glycol). Glycerol is the preferred plasticiser. An alternative preferred plasticiser is the ester derived from boric acid and glycerol. When present, the organic plasticiser may comprise up to about 45% by weight of the hydrogel composition.

### Surfactants

Any compatible surfactant may optionally be used as an additional ingredient of the hydrogel composition. Surfactants can lower the surface tension of the mixture before polymerisation and thus aid processing. Non-ionic, anionic and cationic surfactants are preferred. The surfactant ideally comprises any of the surfactants listed below either alone or in combination with each other and/or with other surfactants. The total amount of surfactant, if present, is suitably up to about 10% by weight of the hydrogel composition, preferably from about 0.05% to about 4% by weight.

### 1. Non-ionic Surfactants

Suitable non-ionic surfactants include, but are not limited to, those selected from the group consisting of the condensation products of a higher aliphatic alcohol, such as a fatty alcohol, containing about 8 to about 20 carbon atoms, in a straight or branched chain configuration, condensed with about 3 to about 100 moles, preferably about 5 to about 40 moles and most preferably about 5 to about 20 moles of ethylene oxide. Examples of such non-ionic ethoxylated fatty alcohol surfactants are the Tergitol™ 15-S series from Union Carbide and Brij™ surfactants from ICI. Tergitol™ 15-S surfactants include C₁₁-C₁₅ secondary alcohol polyethyleneglycol ethers. Brij™ 58 surfactant is polyoxyethylene(20) cetyl ether, and Brij™ 76 surfactant is polyoxyethylene(10) stearyl ether.

Other suitable non-ionic surfactants include, but are not limited to, those selected from the group consisting of the polyethylene oxide condensates of one mole of alkyl phenol containing from about 6 to 12 carbon atoms in a straight or branched chain configuration, with about 3 to about 100 moles of ethylene oxide. Examples of non- ionic surfactants are the Igepal™ CO and CA series from Rhone-Poulenc. Igepal™ CO surfactants include nonylphenoxy poly(ethyleneoxy) ethanols. Igepal™ CA surfactants include octylphenoxy poly(ethyloneoxy) ethanols.

Another group of usable non-ionic surfactants include, but are not limited to, those selected from the group consisting of block copolymers of ethylene oxide and propylene oxide or butylene oxide. Examples of such non-ionic block copolymer surfactants are the Pluronic™ and Tetronic™ series of surfactants from BASF. Pluronic™ surfactants include ethylene oxide-propylene oxide block copolymers. Tetronic™ surfactants include ethylene oxide-propylene oxide block copolymers. The balance of hydrophobic and hydrophilic components within the surfactant together with the molecular weight are found to be important. Suitable examples are Pluronic L68 and Tetronic 1907. Particularly suitable examples are Pluronic L64 and Tetronic 1107.

Still other satisfactory non-ionic surfactants include, but are not limited to, those selected from the group consisting of sorbitan fatty acid esters, polyoxyethylene sorbitan fatty acid esters and polyoxyethylene stearates. Examples of such fatty acid ester non- ionic surfactants are the Span™, Tween™, and Myrj™ surfactants from ICI. Span™ surfactants include C₁₂-C₁₈ sorbitan monoesters. Tween™ surfactants include poly(ethylene oxide) C₁₂-C₁₈ sorbitan monoesters. Myrj™ surfactants include poly(ethylene oxide) stearates.

### 2. Anionic Surfactants

Anionic surfactants normally include a hydrophobic moiety selected from the group consisting of (about C₆ to about C₂₀) alkyl, alkylaryl, and alkenyl groups and an anionic group selected from the group consisting of sulfate, sulfonate, phosphate, polyoxyethylene sulfate, polyoxyethylene sulfonate, polyoxyethylene phosphate and the alkali metal salts, ammonium salts, and tertiary amino salts of such anionic groups.

Anionic surfactants which can be used in the present invention include, but are not limited to. those selected from the group consisting of (about C₆ to about C₂₀) alkyl or alkylaryl sulfates or sulfonates such as sodium lauryl sulfate (commercially available as Polystep™ B-3 from Srepan Co.) and sodium dodecyl benzene sulfonate, (commercially available as Siponate™ DS-10 from Rhone-Poulenc); polyoxyethylene (about C₆ to about C₂₀) alkyl or alkylphenol ether sulfates with the ethylene oxide repeating unit in the surfactant below about 30 units, preferably below about 20 units, most preferably below about 15 units, such as Polystep™ B-1 commercially available from Stepan Co. and Alipal™ EP110 and 115 from Rhone-Poulenc; (about C₆ to about C₂₀) alkyl or alkylphenoxy poly (ethyleneoxy)ethyl mono-esters and di-esters of phosphoric acid and its salts, with the ethylene oxide repeating unit in the surfactant below about 30 units, preferably below about 20 units, most preferably below about 15 units, such as Gafac™ RE-510 and Gafac™ RE-610 from GAF.

### 3. Cationic Surfactants

Cationic surfactants useful in the present invention include, but are not limited to, those selected from the group consisting of quaternary ammonium salts in which at least one higher molecular weight group and two or three lower molecular weight groups are linked to a common nitrogen atom to produce a cation, and wherein the electrically-balancing anion is selected from the group consisting of a halide (bromide, chloride, etc.), acetate, nitrite, and lower alkosulfate (methosulfate etc.). The higher molecular weight substituent(s) on the nitrogen is/are often (a) higher alkyl group(s), containing about 10 to about 20 carbon atoms, and the lower molecular weight substituents may be lower alkyl of about 1 to about 4 carbon atoms, such as methyl or ethyl, which may be substituted, as with hydroxy, in some instances. One or more of the substituents may include an aryl moiety or may be replaced by an aryl, such as benzyl or phenyl.

In a preferred embodiment of the invention the surfactant comprises at least one propylene oxide/ethylene oxide block copolymer, for example such as that supplied by BASF Plc under the trade name Pluronic P65 or L64.

### Other additives

The hydrogel components of the present invention may include one or more additional ingredients, which may be added to the pre-polymerisation mixture or the polymerised product, at the choice of the skilled worker. Such additional ingredients are selected from additives known in the art, including, for example, water, organic plasticisers, surfactants, polymers, electrolytes, pH regulators, colorants, chloride sources, bioactive compounds, personal and body care agents, and mixtures thereof. The polymers can be natural polymers (e.g. xanthan gum), synthetic polymers (e.g. polyoxypropylene-polyoxyethylene block copolymer or poly-(methyl vinyl ether *alt* maleic anhydride)), or any combination thereof. By "bioactive compounds" we mean any compound or mixture included within the hydrogel for some effect it has on living systems as opposed to the hydrogel, whether the living system be bacteria or other microorganisms or higher animals such as the intended user of articles incorporating the hydrogel. A biocidal biaoactive compound that may particularly be mentioned is citric acid.

Additional polymer(s), typically rheology modifying polymer(s), may be incorporated into the polymerisation reaction mixture at levels typically up to about 10% by weight of total polymerisation reaction mixture, e.g. from about 0.2% to about 10% by weight. Such polymer(s) may include polyacrylamide, poly-NaAMPS, polyethylene glycol (PEG), polyvinylpyrrolidone (PVP) or carboxymethyl cellulose.

A particularly preferred application is in the field of biomedical skin electrodes. When the hydrogels are intended for use in conjunction with Ag/AgCl medical electrodes, chloride ions are required to be present in order for the electrode to function. Potassium chloride and sodium chloride are commonly used. However any compound capable of donating chloride ions to the system may be used, for example, lithium chloride, calcium chloride, magnesium chloride or ammonium chloride. The amount that should be added is dependent on the electrical properties required and is typically about 0.5-8% by weight.

In general, an electrolyte (e.g. a salt such as a chloride as mentioned above or another salt such as a nitrate, for example sodium or calcium nitrate) will need to be included in the polymerisation reaction mixture in appropriate amounts, when the process is used to manufacture a hydrogel composition for use in an electrode.

The composite sheets according to the present invention are used in biomedical electrodes in generally conventional manner, as will be readily understood by those skilled in this art. Such biomedical electrodes may include electrodes (suitably in patch form) for diagnostic, stimulation, therapeutic and electrosurgical use. The hydrogel composites according to the present invention will typically provide good current dispersion over the skin-electrode interface, leading to potential benefits through reduction of electrical hot-spots.

Additional functional ingredients may also incorporated in the hydrogel composites used in the invention, including bioactive compounds such as antimicrobial agents (e.g. citric acid, stannous chloride), enzymes, compounds providing a heating or cooling sensation to a patient's body, dermatologically active compounds and, for drug delivery applications, pharmaceutically active agents, the latter being designed to be delivered either passively (e.g. transdermally) or actively (e.g. iontophoretically) through the skin.

For use in pharmaceutical delivery devices for the delivery of pharmaceuticals or other active agents to or through mammalian skin, the composites may optionally contain topical, transdermal or iontophoretic agents and excipients. The composites may contain penetration-enhancing agents to assist the delivery of water or active agents into the skin. Non-limiting examples of penetration-enhancing agents for use in such applications include methyl oleic acid, isopropyl myristate, Azone™, Transcutol™ and N-methyl pyrrolidone.

The additional ingredient may comprise an antimicrobial agent stable against light and radiation, comprising an effective amount of antimicrobial metal (e.g. silver) ions and stabilizing halide (e.g. chloride) ions, wherein the halide is present in an excess (preferably in a substantial molar excess such as around 500-fold excess) with respect to the amount of metal ions.

The hydrogel components of the composites of the present invention preferably consist essentially of a cross-linked hydrophilic polymer of a hydrophilic monomer and optionally one or more comonomer, together with water and/or one or more organic plasticiser, and optionally together with one or more additives selected from surfactants, polymers, pH regulators, electrolytes, chloride sources, bioactive compounds and mixtures thereof, with less than about 10% by weight of other additives.

### Applications

The hydrogel composites described herein may suitably be used in a range of skin contact or covering applications where the composite is brought into contact either with skin or with an intermediary member which interfaces between the composite and the skin. The composite may be unsupported or may be supported on a part of a larger article for some specific use, e.g. a support or backing structure. The materials and articles may suitably be in the form of sheets, coatings or membranes. Applications include patches, tapes, bandages, devices and dressings of general utility or for specific uses, including without limitation biomedical, skin care, personal and body care, palliative and veterinary uses such as, for example, skin electrodes for diagnostic (e.g. ECG), stimulation (e.g. TENS), therapeutic (e.g. defibrillation) or electrosurgical (e.g. electrocauterisation) use; dressings and reservoirs for assisting wound and burn healing, wound and burn management, skin cooling, skin moisturizing, skin warming, aroma release or delivery, decongestant release or delivery, pharmaceutical and drug release or delivery, perfume release or delivery, fragrance release or delivery, scent release or delivery, and other skin contacting devices such as absorbent pads or patches for absorbing body fluids (e.g. lactation pads for nursing mothers), hairpiece adhesives and clothing adhesives; and adhesive flanges and tabs for fecal collection receptacles, ostomy devices and other incontinence devices.

The hydrogel composites in accordance with the present invention enable a high degree of water and aqueous fluid retention. This has considerable advantages in certain important applications, for example in the treatment of wounds and bums (where an aqueous environment is advantageous for wound healing and reduced scarring), and in skin care more generally.

### Brief Description of the Drawings

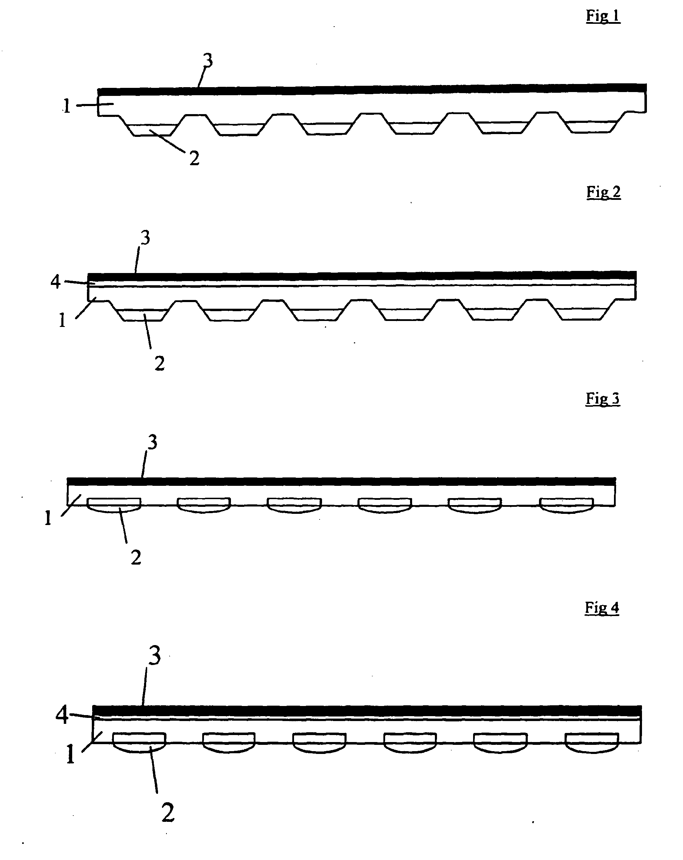

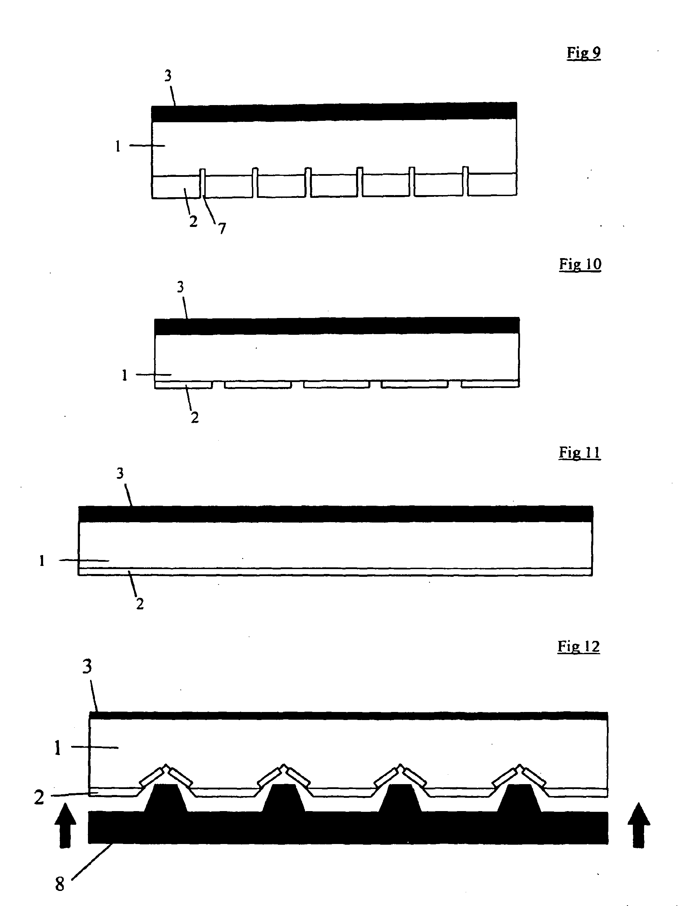

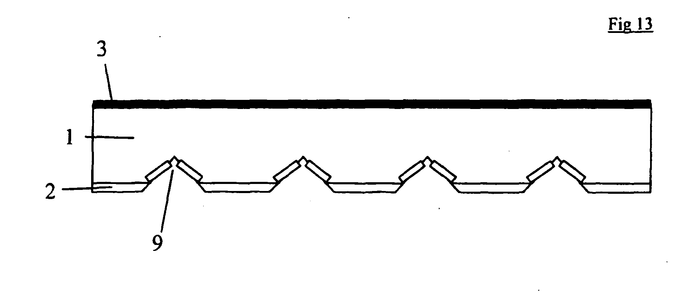

The invention will now be described further, without limitation and purely by way of example, with reference to the accompanying drawings, in which Figures 1 to 10 illustrate in transverse cross-section the internal layer structure of various absorbent hydrogel materials according to the present invention; Figure 11 illustrates in transverse cross-section a starting multi-layer structure from which an alternative absorbent hydrogel material according to the present invention can be formed; Figure 12 illustrates in transverse cross-section the step of forming three-dimensional surface features on the structure of Figure 11; and Figure 13 illustrates in transverse cross-section the absorbent hydrogel material resulting from the step of Figure 12.

### Detailed Description of the Drawings

Referring to the drawings, in each case the absorbent hydrogel layer part of the composite is represented by the numeral 1, the continuous second hydrogel part by the numeral 2, the relatively strong support layer, e.g. of polyurethane film or polyurethane film/foam or other non-hydrogel polymer material, by the numeral 3, and any additional continuous hydrogel layer by the numeral 4.

The illustrated absorbent hydrogel composite sheets consist essentially of:

In Figure 1, layer 1, part 2 and layer 3, with layers 1 and 3 optionally associated together via an intermediate adhesive (not shown). The support layer 3 may optionally (not shown) be of greater dimension that the absorbent hydrogel composite of layer 1 and part 2, in order to create an adhesive bordered overlay, e.g. in a wound or burn dressing. Layer 1 is moulded to create surface features 5 (indentations and ridges), and the hydrogel part 2 is impregnated into the ridge portions of the surface features 5, leaving the absorbent hydrogel material of layer 1 in the indentations effectively exposed to absorb external water from the skin-contacting side (the lower side as drawn). This arrangement is suitably manufactured by letting a liquid pre-gel precursor for 2 infiltrate into the cellular voids of layer 1 in the ridge portions 5, and then curing the pre-gel to form the component 2.

In Figure 2, as for Figure 1, with additionally an additional relatively non-absorbent layer 4 between layers 1 and 3. Layer 4 is preferably infiltrated into cellular voids in the upper portion of layer 1. The additional layer 4 serves to lock away excess moisture absorbed into the absorbent layer 1 (e.g. excess wound exudate when the composite is used in a wound or burn dressing).

In Figure 3, layer 1, part 2 and layer 3, with layers 1 and 3 optionally associated together via an intermediate adhesive (not shown). The support layer 3 may optionally (not shown) be of greater dimension that the absorbent hydrogel composite of layer 1 and part 2, in order to create an adhesive bordered overlay, e.g. in a wound or burn dressing. Hydrogel part 2 is impregnated as islands into discrete portions of the flat skin-contacting side (the lower side as drawn) of layer 1, leaving intermediate regions of the layer 1 effectively exposed to absorb external water from that side. This arrangement is suitably manufactured by letting a liquid pre-gel precursor for 2 infiltrate into the cellular voids of regions of layer 1 on the lower side as drawn, and then curing the pre-gel to form the component part 2.

In Figure 4, as for Figure 3, with additionally an additional relatively non-absorbent layer 4 between layers 1 and 3. Layer 4 is preferably infiltrated into cellular voids in the upper portion of layer 1. The additional layer 4 serves to lock away excess moisture absorbed into the absorbent layer 1 (e.g. excess wound exudate when the composite is used in a wound or burn dressing).

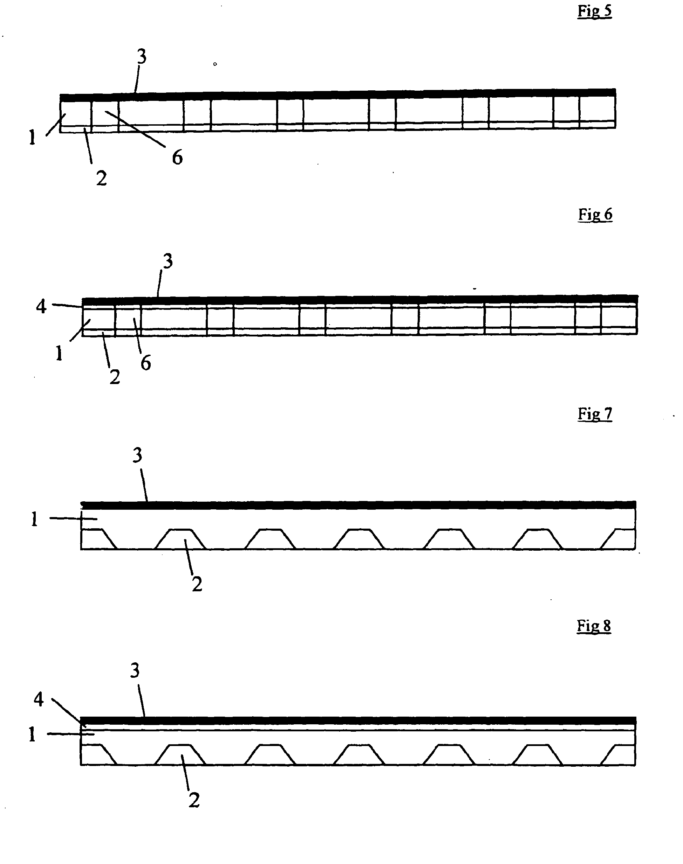

In Figure 5, layer 1, part 2 and layer 3, with layers 1 and 3 optionally associated together via an intermediate adhesive (not shown). The support layer 3 may optionally (not shown) be of greater dimension that the absorbent hydrogel composite of layer 1 and part 2, in order to create an adhesive bordered overlay, e.g. in a wound or burn dressing. The coat weight of the part 2 may suitably be about 200 g/m². The part 2 may optionally contain an internal scrim, as described above. Prior to applying layer 3, hydrogel part 2 is initially infiltrated as a full covering layer over the flat skin-contacting side (the lower side as drawn) of layer 1, and apertures 6 are cut fully through the layers 1 and 2 to create a "foam net", leaving the internal walls of the apertures in the interior of layer 1 effectively exposed to absorb external water from the skin-contacting side. After the through-apertures 6 have been formed, the layer 3 is applied into association with the upper side of layer 1.

In Figure 6, as for Figure 5, with additionally an additional relatively non-absorbent layer 4 between layers 1 and 3. Layer 4 is preferably infiltrated into cellular voids in the upper portion of layer 1, prior to cutting the aperture, so that the apertures 6 penetrate fully through layers 1, 2 and 4. The additional layer 4 serves to lock away excess moisture absorbed into the absorbent layer 1 (e.g. excess wound exudate when the composite is used in a wound or burn dressing).

In Figure 7, layer 1, part 2 and layer 3, with layers 1 and 3 optionally associated together via an intermediate adhesive (not shown). The support layer 3 may optionally (not shown) be of greater dimension that the absorbent hydrogel composite of layer 1 and part 2, in order to create an adhesive bordered overlay, e.g. in a wound or burn dressing. Layer 1 is moulded to create surface features 5' (indentations), and the hydrogel part 2 is impregnated and filled (e.g. poured) into the indentations 5', providing a flat skin-contacting side (the lower side as drawn) and leaving the absorbent hydrogel material of layer 1 between the indentations effectively exposed to absorb external water from that skin-contacting side. This arrangement is suitably manufactured by letting a liquid pre-gel precursor for 2 infiltrate into the cellular voids of layer 1 in the indentations 5', as well as fill the indentations 5', and then curing the pre-gel to form the component 2.

In Figure 8, as for Figure 7, with additionally an additional relatively non-absorbent layer 4 between layers 1 and 3. Layer 4 is preferably infiltrated into cellular voids in the upper portion of layer 1. The additional layer 4 serves to lock away excess moisture absorbed into the absorbent layer 1 (e.g. excess wound exudate when the composite is used in a wound or burn dressing).

In Figure 9, layer 1, part 2 and layer 3, with layers 1 and 3 optionally associated together via an intermediate adhesive (not shown). The support layer 3 may optionally (not shown) be of greater dimension that the absorbent hydrogel composite of layer 1 and part 2, in order to create an adhesive bordered overlay, e.g. in a wound or burn dressing. The coat weight of the part 2 may suitably be about 200 g/m². The part 2 may optionally contain an internal scrim, as described above. Slits 7, optionally linear, curved or a combination, and optionally parallel, non-parallel, randomly oriented, intersecting or any combination thereof (e.g. crosses), are provided, through part 2 and extending some way into layer 1, leaving the internal walls of the slits 7 in the interior of layer 1 effectively exposed to absorb external water from the skin-contacting side (the lower side as drawn). The slits facilitate the passage of wound exudate or other fluids into the layer 1. This arrangement is suitably manufactured by letting a liquid pre-gel precursor for 2 infiltrate into the cellular voids of regions of layer 1 on the lower side as drawn, and then curing the pre-gel to form the part 2 as a layer over substantially the entire lower side of the layer 1, and then cutting the slits 7 in conventional manner using a cutting apparatus.

In Figure 10, layer 1, part 2 and layer 3, with layers 1 and 3 optionally associated together via an intermediate adhesive (not shown). The support layer 3 may optionally (not shown) be of greater dimension that the absorbent hydrogel composite of layer 1 and part 2, in order to create an adhesive bordered overlay, e.g. in a wound or burn dressing. The coat weight of the part 2 where it is applied may suitably be about 200 g/m². The part 2 may optionally contain an internal scrim, as described above. Hydrogel part 2 is impregnated as islands into discrete portions of the flat skin-contacting side (the lower side as drawn) of layer 1, leaving intermediate openings or apertures which cause the regions of the layer 1 at those places to be effectively exposed to absorb external water from that side. The openings or apertures facilitate the passage of wound exudate or other fluids into the layer 1. This arrangement is suitably manufactured by printing a liquid pre-gel precursor for 2 onto the relevant face of the layer 1 (the lower side as drawn) so as to infiltrate into the cellular voids of regions of layer 1, and then curing the pre-gel to form the component 2.

Figure 11 illustrates in transverse cross-section a starting multi-layer structure from which an alternative absorbent hydrogel material according to the present invention can be formed. This comprises layer 1, part 2 as a layer over substantially the entire lower side of the layer 1 as drawn (the skin-contacting side) and layer 3, with layers 1 and 3 optionally associated together via an intermediate adhesive (not shown). The coat weight of the part 2 may suitably be about 200 g/m². The part 2 may optionally contain an internal scrim, as described above. This arrangement is suitably manufactured by letting a liquid pre-gel precursor for 2 infiltrate into the cellular voids of regions of layer 1 on the lower side as drawn, optionally with the scrim if desired, and then curing the pre-gel to form the part 2 as a layer over substantially the entire lower side of the layer 1 Alternatively, part 2, optionally encapsulating with the scrim if desired, can be pre-formed as a layer and laminated to the lower side of layer 1 as drawn, in conventional manner.

Figure 12 illustrates in transverse cross-section the step of forming three-dimensional surface features on the structure of Figure 11. A conventional die 8 punches through the layer of hydrogel 2 from the side which is to form the skin-contacting side of the finished product (the lower side as drawn), moving in the direction of the arrows. This punching can optionally take place at the same time as the shape of the desired final article is cut in the sheet. The die 8 is then withdrawn in the reverse direction.

Figure 13 illustrates in transverse cross-section the absorbent hydrogel material resulting from the step of Figure 12 after withdrawal of the die. Apertures 9 have been punched through the hydrogel part 2, typically with some local deformation of the surface around the apertures 9. These apertures 9 cause the underlying regions of the layer 1 at those places to be effectively exposed to absorb external water from the skin-contacting side. The openings or apertures facilitate the passage of wound exudate or other fluids into the layer 1.

### Examples

The invention will be further described with reference to the following Examples, which should not be understood to limit the scope of the invention.

### Example 1

A precursor solution comprising 70 parts by weight of a 58% aqueous solution of the sodium salt of acrylamidomethylpropanesulphonic acid (NaAMPS, LZ2405 Lubrizol), 30 parts glycerol and 0.14 parts of a 1 to 10 (by weight) mixture of Daracure 1173 photoinitiator (Ciba Speciality Chemicals) and IRR280 cross-linker (PEG400 diacrylate, UCB Chemicals) was dispensed from a slot die 120mm wide at a coat weight of 200g/m² onto a moving web of a 562B medical grade hydrophilic foam sample from Rynel supported on a web of siliconised paper (Cotek) moving at 7m/s and cured with a NUVA Solo 30 medium pressure mercury arc lamp (GEW). A 1 ml drop of saline was absorbed in less than 5s. Larger volumes of liquid for example 5 to 10 ml absorbed into 40cm² of the above hydrogel/foam composite is largely held within the structure such that the liquid cannot be squeezed out. This is not the case when 5 to 10 ml of saline is absorbed into the foam only.

### Example 2

A precursor solution comprising 70 parts by weight of a 58% aqueous solution of the sodium salt of acrylamidomethylpropanesulphonic acid (NaAMPS, LZ2405 Lubrizol), 30 parts glycerol and 0.14 parts of a 1 to 10 (by weight) mixture of Daracure 1173 photoinitiator (Ciba Speciality Chemicals) and IRR280 cross-linker (PEG400 diacrylate, UCB Chemicals) was dispensed from a slot die 120mm wide at a coat weight of 450g/m² onto a moving web of a 562B medical grade hydrophilic foam sample from Rynel supported on a web of siliconised paper (Cotek) moving at 7m/s and cured with a NUVA Solo 30 medium pressure mercury arc lamp (GEW). A 1 ml drop of saline was absorbed in less than 5s. Larger volumes of liquid for example 5 to 10 ml absorbed into 40cm² of the above hydrogel/foam composite is largely held within the structure such that the liquid cannot be squeezed out. This is not the case when 5 to 10 ml of saline is absorbed into the foam only.

### Industrial Applicability

The present invention makes available absorbent hydrogels with useful capacity to absorb potentially large quantities of liquids at an acceptable speed for many uses. Moreover, the hydrogels can be made conveniently and efficiently.

## Claims

1. An absorbent material or article comprising an absorbent hydrogel composite sheet including: a first hydrophilic absorbent layer part having two major faces and having an internal cellular structure and a second part which comprises a flexible plasticised hydrophilic polymer matrix having an internal structure which is relatively continuous in relation to the internal structure of the first part, provided that at least one of the said first part and the said second part comprises a hydrogel; wherein the first part and the second part arc not formed integrally together in one step from a single pre-gel mixture and the second part is associated with the layer part such that the second part ether:
(a) infiltrates some but not all of the cellular voids of the first hydrophilic absorbent layer part over a first major face of the absorbent layer part; or
(b) overlies a major face of the absorbent layer part whereby some but not all of the first part is effectively exposed to permit absorption of external water or other fluid into the cellular voids through the face of the sheet corresponding to that said major face of the absorbent layer.

2. An absorbent material or article according to claim 1, wherein there is provided over the other major face of the layer part a support layer, e.g. of a polymeric material.

3. An absorbent material or article according to claim 2, wherein the support layer is permeable to moisture liquid and/or moisture vapour.

4. An absorbent material or article according to claim 2, wherein the support layer is impermeable to moisture liquid and/or moisture vapour.

5. An absorbent material or article according to claim 2, wherein the support layer has regions which are permeable to moisture liquid and/or moisture vapour and regions which are impermeable to moisture liquid and/or moisture vapour.

6. An absorbent material or article according to any one of claims 2 to 5, wherein the support layer is a polymeric supporting sheet or film.

7. An absorbent material or article according to any one of claims 2 to 6, wherein the support layer comprises a non-hydrogel polymer.

8. An absorbent material or article according to any one of claims 2 to 7, comprising one or more additional layer between the first, part and the support layer.

9. An absorbent material or article according to any one of claims 2 to 8, wherein the support layer is formed of a polymer or a fabric.

10. An absorbent material or article according to any one of claims 2 to 9, wherein the support layer has a web, film, sheet or net structure or any combination thereof.

11. An absorbent material or article according to any one of the preceding claims, wherein the first part comprises a flexible plasticised hydrophilic polymer matrix.

12. An absorbent material or article according to claim 11, wherein the flexible plasticised hydrophilic polymer matrix materials forming the said first part and second part of the absorbent hydrogel composite sheet are different.

13. An absorbent material or article according to claim 11, wherein the flexible plasticised hydrophilic polymer matrix materials forming the said first part and second part of the absorbent hydrogel composite sheet are the same.

14. An absorbent material or article according to any one of the preceding claims, wherein the first part comprises a hydrophilic polyurethane foam.

15. An absorbent material or article according to any one of the preceding claims, wherein the first part comprises a hydrogel.

16. An absorbent material or article according to any one of the preceding claims, wherein the second part comprises a hydrogel.

17. An absorbent material or article according to claim 14, wherein the hydrogel infiltrates some but not all cellular voids of the first part.

18. An absorbent material or article according to any one of the preceding claims, wherein the weight of the second part is up to 500 g/m².

19. An absorbent material or article according to claim 16, wherein the weight of the second part is between 50 and 460 g/m².

20. An absorbent material or article according to claim 17, wherein the weight of the second part is 200 g/m².

21. An absorbent material or article according to any one of the preceding claims, wherein the first part comprises a porous foam having an internal cellular structure such that the volume ratio of cell void to matrix is greater than 1:3 and the second part has a volume ratio of cell void to matrix of less than 1:20.

22. An absorbent material or article according to any one of the preceding claims, consisting essentially of the first part, the second part disposed on some but not all of one major face of the first part and a support layer adhered to the other major face of the first part.

23. An absorbent material or article according to any one of claims 1 to 21, consisting essentially of the first part, the second part disposed on some but not all of one major face of the first part, a support layer adhered to the other major face of the first part and an additional continuous hydrogel layer disposed between the first part and the support layer.

24. An absorbent material or article according to any one of claims 1 to 21, consisting essentially of the first part, the second part disposed on all of one major face of the first part and a support layer adhered to the other major face of the first part, with apertures penetrating the second part and the full thickness of the first part up to, but not including, the support layer.

25. An absorbent material or article according to any one of claims 1 to 21, consisting essentially of the first part, the second part disposed on all of one major face of the first part a support layer adhered to the other major face of the first part, with apertures penetrating the second part and the full thickness of the first part, up to, but not including, the support layer and an additional continuous hydrogel layer disposed between the first part and the support layer, the apertures penetrating also the additional continuous hydrogel layer.

26. An absorbent material or article according to any one of claims 1 to 21, consisting essentially of the first part, and the second part disposed in depressions provided in one major face of the first part so that the second part covers some but not all of that major face of the first part and a support layer adhered to the other major face of the first part.

27. An absorbent material or article according to any one of claims 1 to 21, consisting essentially of the first part, and the second part disposed in depressions provided in one maj or face of the first part so that the second part covers some but not all of that major face of the first part, a support layer adhered to the other major face of the first part, and an additional continuous hydrogel layer disposed between the first part and the support layer.

28. An absorbent material or article according to any one of claims 1 to 21, consisting essentially of the first part, the second part printed on some but not all of one major face of the first part and a support layer adhered to the other major face of the first part.

29. An absorbent material or article according to any one of claims 1 to 21, consisting essentially of the first part, the second part printed on some but not all of one major face of the first part, a support layer adhered to the other major face of the first part, and an additional continuous hydrogel layer disposed between the first part and the support layer.

30. A process for the preparation of an absorbent hydrogel composite sheet, comprising associating a first hydrophilic absorbent layer, part having two major faces and having an internal cellular structure and second part which comprises a flexible plasticised hydrophilic polymer matrix having an internal structure which is relatively continuous in relation to the internal structure of the first part, provided that at least one of the said first part and the said second part comprises a hydrogel such that the second part either.
(a) infiltrates some but not all of the cellular voids of the first hydrophilic absorbent layer part over a first major face of the absorbent layer part; and/or b) overlies a major face of the absorbent layer part
whereby some but not all of the first part is effectively exposed to permit absorption of external water or other fluid into the cellular voids through the face of the sheet corresponding to that said major face of the absorbent layer, and wherein the first part and the second part are not formed integrally together in one step from a single pre-gel mixture.

31. A process according to claim 30 further comprising associating the first part and second part of the hydrogel composite sheet with at least one other layer or component before, during or after the association of the first part and the second parts of the hydrogel composite sheet.

32. A process according to claim 31, wherein the at least one other layer or component includes a support layer.

33. A process according to any one of claims 30 to 32, wherein the association of the parts is achieved by:
(a) forming the first part of the composite as a foam layer in the solid cured state;
(b) applying to a surface thereof a liquid pre-gel precursor for the second part of the composite;
(c) allowing the liquid pre-gel precursor to infiltrate the pores of the first part to a desired extent; and
(d) subsequently curing the second part to provide the composite sheet.

34. A process according to any one of claims 30 to 32, wherein the association of the parts is achieved by adhering or laminating in a conventional manner pre-formed areas of the second part of the composite to a pre-formed layer part of the composite.

35. A process according to any one of claims 30 to 34 wherein the absorbent hydrogel composite sheet is for use in the absorbent material or article according to any one of the preceding claims.

36. An absorbent material or article comprising an absorbent hydrogel composite sheet made by the process according to any one of claims 30 to 34.

37. A biomedical electrode comprising an electrically conductive current distribution member adapted for electrical connection to an electrical apparatus, and an electrically conductive skin contactable portion in association with the current distribution member, whereby electrical current can flow between the electrical apparatus and a wearer's skin when the electrode is in use, wherein the electrically conductive skin contactable portion comprises an absorbent material or article according to any one of claims 1 to 29 or 36.

38. A wound or burn dressing comprising an absorbent member adapted to contact a wearer's skin in the location of a wound or burn, and a sheet backing member supporting the absorbent member, the sheet backing member including a portion which extends beyond the absorbent member and defines a skin-directed surface which carries a pressure-sensitive adhesive for securement of the dressing to the wearer's skin, wherein the absorbent member comprises an absorbent hydrogel composite or a hydrogel/release layer combination according to any one of claims 1 to 29 or 36.

## Patentansprüche

1. Absorbierendes Material oder absorbierender Gegenstand umfassend eine absorbierende Hydrogelverbundstoffschicht beinhaltend: als einen ersten Anteil eine hydrophile, absorbierende Lage mit zwei Hauptflächen und einer zellenförmigen inneren Struktur und einen zweiten Anteil, der eine flexible, plastifizierte, hydrophile Polymatrix mit einer inneren Struktur umfasst, die in Bezug auf die innere Struktur des ersten Anteils verhältnismäßig durchgängig ist, mit der Maßgabe, dass mindestens einer des ersten und des zweiten Anteils ein Hydrogel enthält, worin der erste und der zweite Anteil nicht in ganzheitlicher Weise zusammen in einem Schritt aus einer einzigen Prägelmischung gebildet werden und der zweite Anteil mit dem Lagen-Anteil in der Weise verbunden ist, dass der zweite Anteil entweder
a) einige, aber nicht alle zellenförmigen Hohlräume des ersten hydrophilen, absorbierenden Lagen-Anteils über eine erste Hauptfläche des absorbierenden Lagen-Anteils hinweg infiltriert oder
b) eine Hauptfläche des absorbierenden Lagen-Anteils überlagert, wodurch einige, aber nicht alle Bereiche des ersten Anteils effektiv exponiert sind, um die Absorption äußeren Wassers oder eines anderen Fluids durch die Fläche der der Hauptfläche der absorbierenden Lage entsprechenden Schicht hindurch in die zellenförmigen Hohlräume zu gestatten.

2. Absorbierendes Material oder absorbierender Gegenstand nach Anspruch 1, worin eine Stützschicht, z.B. aus einem polymeren Material, über die andere Hauptfläche des Lagen-Anteils hinweg bereitgestellt wird.

3. Absorbierendes Material oder absorbierender Gegenstand nach Anspruch 2, worin die Stützschicht für flüssige und/oder wasserdampfförmige Feuchtigkeit durchlässig ist.

4. Absorbierendes Material oder absorbierender Gegenstand nach Anspruch 2, worin die Stützschicht für flüssige und/oder wasserdampfförmige Feuchtigkeit undurchlässig ist.

5. Absorbierendes Material oder absorbierender Gegenstand nach Anspruch 2, worin die Stützschicht Bereiche hat, die für flüssige und/oder wasserdampfförmige Feuchtigkeit durchlässig sind und Bereiche, die für flüssige und/oder wasserdampfförmige Feuchtigkeit undurchlässig sind.

6. Absorbierendes Material oder absorbierender Gegenstand nach einem der Ansprüche 2 bis 5, worin die Stützschicht ein(e) Polymerstützschicht oder -film ist.

7. Absorbierendes Material oder absorbierender Gegenstand nach einem der Ansprüche 2 bis 6, worin die Stützschicht ein Polymer umfasst, das kein Hydrogel ist.

8. Absorbierendes Material oder absorbierender Gegenstand nach einem der Ansprüche 2 bis 7, umfassend eine oder mehrere zusätzliche Lagen zwischen dem ersten Anteil und der Stützschicht.

9. Absorbierendes Material oder absorbierender Gegenstand nach einem der Ansprüche 2 bis 8, worin die Stützschicht aus einem Polymer oder einem Gewebe gebildet ist.

10. Absorbierendes Material oder absorbierender Gegenstand nach einem der Ansprüche 2 bis 9, worin die Stützschicht eine Matrix-, Film-, Schicht- oder Netzstruktur oder jedwede Kombination davon hat.

11. Absorbierendes Material oder absorbierender Gegenstand nach einem der vorangehenden Ansprüche, worin der erste Anteil eine flexible, plastifizierte, hydrophile Polymermatrix umfasst.

12. Absorbierendes Material oder absorbierender Gegenstand nach Anspruch 11, worin die den ersten und den zweiten Anteil der absorbierenden Hydrogelverbundstoffschicht bildenden, flexiblen, plastifizierten, hydrophilen Polymermatrix-Materialien verschieden sind.

13. Absorbierendes Material oder absorbierender Gegenstand nach Anspruch 11, worin die den ersten und den zweiten Anteil der absorbierenden Hydrogelverbundstoffschicht bildenden, flexiblen, plastifizierten, hydrophilen Polymermatrix-Materialien gleich sind.

14. Absorbierendes Material oder absorbierender Gegenstand nach einem der vorangehenden Ansprüche, worin der erste Anteil einen hydrophilen Polyurethanschaum umfasst.

15. Absorbierendes Material oder absorbierender Gegenstand nach einem der vorangehenden Ansprüche, worin der erste Anteil ein Hydrogel umfasst.

16. Absorbierendes Material oder absorbierender Gegenstand nach einem der vorangehenden Ansprüche, worin der zweite Anteil ein Hydrogel umfasst.

17. Absorbierendes Material oder absorbierender Gegenstand nach Anspruch 14, worin das Hydrogel einige, aber nicht alle zellenförmigen Hohlräume des ersten Anteils infiltriert.

18. Absorbierendes Material oder absorbierender Gegenstand nach einem der vorangehenden Ansprüche, worin das Gewicht des zweiten Anteils bis zu 500 g/cm² ist.

19. Absorbierendes Material oder absorbierender Gegenstand nach Anspruch 16, worin das Gewicht des zweiten Anteils zwischen 50 und 460 g/cm² ist.

20. Absorbierendes Material oder absorbierender Gegenstand nach Anspruch 17, worin das Gewicht des zweiten Anteils 200 g/cm² ist.

21. Absorbierendes Material oder absorbierender Gegenstand nach einem der vorangehenden Ansprüche, worin der erste Anteil einen porösen Schaum mit einer inneren zellenförmigen Struktur umfasst, so dass das Volumenverhältnis von Zellhohlraum zu Matrix größer als 1:3 ist und der zweite Anteil ein Volumenverhältnis von Zellhohlraum zu Matrix von weniger als 1:20 hat.

22. Absorbierendes Material oder absorbierender Gegenstand nach einem der vorangehenden Ansprüche, im Wesentlichen bestehend aus dem ersten Anteil, dem auf einigen, aber nicht allen Bereichen der einen Hauptfläche des ersten Anteils angeordneten zweiten Anteil und einer an die andere Hauptfläche des ersten Anteils angehafteten Stützschicht.

23. Absorbierendes Material oder absorbierender Gegenstand nach einem der Ansprüche 1 bis 21, im Wesentlichen bestehend aus dem ersten Anteil, dem auf einigen, aber nicht allen Bereichen der einen Hauptfläche des ersten Anteils angeordneten zweiten Anteil, der an die andere Hauptfläche des ersten Anteils angehafteten Stützschicht und einer zusätzlichen, durchgängigen Hydrogelschicht, die zwischen dem ersten Anteil und der Stützschicht angeordnet ist.

24. Absorbierendes Material oder absorbierender Gegenstand nach einem der Ansprüche 1 bis 21, im Wesentlichen bestehend aus dem ersten Anteil, dem auf einigen, aber nicht allen Bereichen der einen Hauptfläche des ersten Anteils angeordneten zweiten Anteil und der an die andere Hauptfläche des ersten Anteils angehafteten Stützschicht mit Öffnungen, die den zweiten Anteil und die gesamte Dicke des ersten Teils bis zu der, aber nicht einschließlich der Stützschicht durchdringen.

25. Absorbierendes Material oder absorbierender Gegenstand nach einem der Ansprüche 1 bis 21, im Wesentlichen bestehend aus dem ersten Anteil, dem auf einigen, aber nicht allen Bereichen der einen Hauptfläche des ersten Anteils angeordneten zweiten Anteil, einer an die andere Hauptfläche des ersten Anteils angehafteten Stützschicht mit Öffnungen, die den zweiten Anteil und die gesamte Dicke des ersten Teils bis zu der, aber nicht einschließlich der Stützschicht durchdringen und einer zusätzlichen durchgängigen, zwischen dem ersten Anteil und der Stützschicht angeordneten Hydrogelschicht, wobei die Öffnungen die zusätzliche durchgängige Hydrogelschicht ebenfalls durchdringen.

26. Absorbierendes Material oder absorbierender Gegenstand nach einem der Ansprüche 1 bis 21, im Wesentlichen bestehend aus dem ersten Anteil und dem zweiten Anteil, der in auf der einen Hauptfläche des ersten Anteils bereitgestellten Vertiefungen so angeordnet ist, dass der zweite Anteil einige, aber nicht alle Bereiche der Hauptfläche des ersten Anteils bedeckt und einer an die andere Hauptfläche des ersten Anteils angehafteten Stützschicht.

27. Absorbierendes Material oder absorbierender Gegenstand nach einem der Ansprüche 1 bis 21, im Wesentlichen bestehend aus dem ersten Anteil und dem zweiten Anteil, der in auf der einen Hauptfläche des ersten Anteils bereitgestellten Vertiefungen so angeordnet ist, dass der zweite Anteil einige, aber nicht alle Bereiche der ersten Hauptfläche bedeckt, einer an die andere Hauptfläche des ersten Anteils angehafteten Stützschicht und einer zusätzlichen, durchgängigen, zwischen dem ersten Anteil und der Stützschicht angeordneten Hydrogelschicht.

28. Absorbierendes Material oder absorbierender Gegenstand nach einem der Ansprüche 1 bis 21, im Wesentlichen bestehend aus dem ersten Anteil, dem auf einigen, aber nicht allen Bereichen einer Hauptfläche des ersten Anteils aufgedruckten zweiten Anteil und einer an die andere Hauptfläche des ersten Anteils angehafteten Stützschicht.

29. Absorbierendes Material oder absorbierender Gegenstand nach einem der Ansprüche 1 bis 21, im Wesentlichen bestehend aus den ersten Anteil, dem auf einigen, aber nicht allen Bereiche der einen Hauptfläche des ersten Anteils aufgedruckten zweiten Anteil, einer an die andere Hauptfläche des ersten Teils angehafteten Stützschicht und einer zusätzlichen, durchgängigen, zwischen dem ersten Anteil und der Stützschicht angeordneten Hydrogelschicht.

30. Verfahren zur Herstellung einer absorbierenden Hydrogelverbundstoffschicht, umfassend das in Verbindung bringen einer hydrophilen, absorbierenden Schicht mit zwei Hauptflächen und einer inneren zellenförmigen Struktur als ersten Anteil und eines zweiten Anteils, der eine flexible, plastifizierte, hydrophile Polymermatrix mit einer inneren Struktur umfasst, die in Bezug auf die innere Struktur des ersten Anteils verhältnismäßig durchgängig ist, mit der Maßgabe, dass mindestens einer des ersten und des zweiten Anteils ein Hydrogel umfasst, so dass der zweite Anteil entweder
a) einige, aber nicht alle zellenförmigen Hohlräume des ersten hydrophilen, absorbierenden Lagen-Anteils über eine erste Hauptfläche des absorbierenden Lagen-Anteils hinweg infiltriert oder
b) eine Hauptfläche des absorbierenden Lagen-Anteils überlagert, wodurch einige, aber nicht alle Bereiche des ersten Anteils effektiv exponiert sind, um die Absorption äußeren Wassers oder eines anderen Fluids durch die Fläche der der Hauptfläche der absorbierenden Lage entsprechenden Schicht hindurch in die zellenförmigen Hohlräume zu gestatten und worin der erste und der zweite Anteil nicht in ganzheitlicher Weise zusammen in einem Schritt aus einer einzigen Prägelmischung gebildet werden.

31. Verfahren nach Anspruch 30, weiterhin umfassend das in Verbindung bringen des ersten und des zweiten Anteils der Hydrogelverbundstoffschicht mit mindestens einer weiteren Schicht oder Komponente bevor, während oder nach dem in Verbindung bringen des ersten und des zweiten Anteils der Hydrogelverbundstoffschicht.

32. Verfahren nach Anspruch 31, worin die mindestens eine weitere Schicht oder Komponente eine Stützschicht beinhaltet.

33. Verfahren nach einem der Ansprüche 30 bis 32, worin die Verbindung der Anteile erreicht wird durch:
(a) Bilden eines ersten Anteils des Verbundstoffs als Schaumschicht in fest ausgehärtetem Zustand,
(b) Auftragen einer flüssigen Prägelvorstufe für den zweiten Anteil des Verbundstoffs auf die Oberfläche davon,
(c) Ermöglichen der Prägelvorstufe zur Infiltrierung der Poren des ersten Anteils bis zu einem gewünschten Ausmaß, und
(d) anschließendes Aushärten des zweiten Anteils zum Bereitstellen der Verbundschicht.

34. Verfahren nach einem der Ansprüche 30 bis 32, worin die Verbindung der Anteile durch Anhaften oder Laminieren von in auf konventionelle Weise vorgefertigten Bereichen des zweiten Verbundstoffs an einen vorgeformten Schicht-Anteil des Verbundstoffs erzielt wird.

35. Verfahren nach einem der Ansprüche 30 bis 34, worin die absorbierende Hydrogelverbundstoffschicht zur Verwendung bei dem absorbierenden Material oder Gegenstand nach einem der vorangehenden Ansprüche bestimmt ist.

36. Absorbierendes Material oder absorbierender Gegenstand umfassend eine durch das Verfahren nach einem der Ansprüche 30 bis 34 hergestellte absorbierende Hydrogelverbundstoffschicht.

37. Biomedizinische Elektrode umfassend ein für die elektrische Verbindung mit einem elektrischen Gerät angepasstes elektrisch leitendes Stromverteilerelement und ein elektrisch leitender, mit Haut in Verbindung mit dem Stromverteilungselement kontaktfähiger Abschnitt, wodurch bei Benutzung der Elektrode elektrischer Strom zwischen dem elektrischen Gerät und der Haut eines Trägers fließen kann, worin der elektrisch leitende, mit Haut kontaktfähige Abschnitt ein absorbierendes Material oder einen absorbierenden Gegenstand nach einem der Ansprüche 1 bis 29 oder 36 umfasst.

38. Wund- oder Verbrennungsverband umfassend ein zum Kontakt bringen mit der Haut eines Trägers an der Stelle einer Wunde oder einer Verbrennung angepasstes absorbierendes Element und ein das absorbierende Element stützendes Schichtverstärkungselement, wobei das Schichtverstärkungselement einen Abschnitt beinhaltet, der sich über das absorbierende Element hinaus erstreckt und eine auf die Haut gerichtete Oberfläche festlegt, die ein druckempfindliches Klebemittel zur Sicherung des Verbands auf der Haut des Trägers trägt, worin das absorbierende Element einen absorbierenden Hydrogelverbundstoff oder eine Kombination aus Hydrogel/Freisetzungs-Schicht nach einem der Ansprüche 1 bis 29 und 36 enthält.

## Revendications

1. Matériau ou article absorbant comprenant une feuille composite d'hydrogel absorbante incluant une première partie couche absorbante hydrophile ayant deux faces principales et ayant une structure cellulaire interne et une deuxième partie comprenant une matrice polymère hydrophile plastifiée souple ayant une structure interne qui est relativement continue par rapport à la structure interne de la première partie, à condition qu'au moins une de ladite première partie et de ladite deuxième partie comprenne un hydrogel ; où la première partie et la deuxième partie ne sont pas intégralement formées ensemble en une étape à partir d'un mélange unique de pré-gel et la deuxième partie est associée à la partie couche de sorte que la deuxième partie :
(a) s'infiltre dans certains mais tous les vides cellulaires de la première partie couche absorbante hydrophile sur une première face principale de la partie couche absorbante ; ou
(b) recouvre une face principale de la partie couche absorbante où une partie mais pas toute la première partie est en fait exposée pour permettre l'absorption d'eau externe ou d'un autre liquide dans les vides cellulaires par la face de la feuille correspondant à ladite face principale de la couche absorbante.

2. Matériau ou article absorbant selon la revendication 1, dans lequel une couche support, par exemple un matériau polymère, est placé sur l'autre face principale de la partie couche.

3. Matériau ou article absorbant selon la revendication 2, dans lequel la couche support est perméable à un liquide humide et/ou à une vapeur humide.

4. Matériau ou article absorbant selon la revendication 2, dans lequel la couche support est imperméable à un liquide humide et/ou à une vapeur humide.

5. Matériau ou article absorbant selon la revendication 2, dans lequel la couche support a des zones qui sont perméables à un liquide humide et/ou à une vapeur humide et des zones qui sont imperméables à un liquide humide et/ou à une vapeur humide.

6. Matériau ou article absorbant selon l'une quelconque des revendications 2 à 5, dans lequel la couche support est une feuille ou un film de support polymère.

7. Matériau ou article absorbant selon l'une quelconque des revendications 2 à 6, dans lequel la couche support comprend un polymère qui n'est pas un hydrogel.

8. Matériau ou article absorbant selon l'une quelconque des revendications 2 à 7, comprenant une ou plusieurs couches supplémentaires entre la première partie et la couche support.

9. Matériau ou article absorbant selon l'une quelconque des revendications 2 à 8, dans lequel la couche support est constituée d'un polymère ou d'un tissu.

10. Matériau ou article absorbant selon l'une quelconque des revendications 2 à 9, dans lequel la couche support a une structure de toile, film, feuille ou filet ou une de leurs combinaisons.

11. Matériau ou article absorbant selon l'une quelconque des revendications précédentes, dans lequel la première partie comprend une matrice polymère hydrophile, plastifiée souple.

12. Matériau ou article absorbant selon la revendication 11, dans lequel les matériaux de la matrice polymère hydrophile plastifiée souple formant la première partie et la deuxième partie de la feuille composite d'hydrogel absorbante sont différents.

13. Matériau ou article absorbant selon la revendication 11, dans lequel les matériaux de la matrice polymère hydrophile plastifiée souple formant la première partie et la deuxième partie de la feuille composite d'hydrogel absorbante sont identiques.

14. Matériau ou article absorbant selon l'une quelconque des revendications précédentes, dans lequel la première partie comprend une mousse polyuréthane hydrophile.

15. Matériau ou article absorbant selon l'une quelconque des revendications précédentes, dans lequel la première partie comprend un hydrogel.

16. Matériau ou article absorbant selon l'une quelconque des revendications précédentes, dans lequel la deuxième partie comprend un hydrogel.

17. Matériau ou article absorbant selon la revendication 14, dans lequel l'hydrogel s'infiltre dans certains mais pas tous les vides cellulaires de la première partie.

18. Matériau ou article absorbant selon l'une quelconque des revendications précédentes, dans lequel le poids de la deuxième partie est inférieur ou égal à 500 g/m².

19. Matériau ou article absorbant selon la revendication 16, dans lequel le poids de la deuxième partie est compris dans la plage allant de 50 à 460 g/m².

20. Matériau ou article absorbant selon la revendication 17, dans lequel le poids de la deuxième partie est de 200 g/m².

21. Matériau ou article absorbant selon l'une quelconque des revendications précédentes, dans lequel la première partie comprend une mousse poreuse ayant une structure cellulaire interne de sorte que le rapport en volume des vides cellulaires à la matrice est supérieur à 1:3 et la deuxième partie a un rapport en volume des vides cellulaires à la matrice inférieur à 1:20.

22. Matériau ou article absorbant selon l'une quelconque des revendications précédentes, comprenant essentiellement la première partie, la deuxième partie placée sur une partie mais pas toute une face principale de la première partie et une couche support adhérant à l'autre face principale de la première partie.

23. Matériau ou article absorbant selon l'une quelconque des revendications 1 à 21, comprenant essentiellement la première partie, la deuxième partie placée sur une partie mais pas toute une face principale de la première partie, une couche support adhérant à l'autre face principale de la première partie et une couche d'hydrogel continue additionnelle placée entre la première partie et la couche support.

24. Matériau ou article absorbant selon l'une quelconque des revendications 1 à 21, comprenant essentiellement la première partie, la deuxième partie placée sur toute une face principale de la première partie et une couche support adhérant à l'autre face principale de la première partie, avec des orifices perçant la deuxième partie et toute l'épaisseur de la première partie jusqu'à, mais sans inclure, la couche support.

25. Matériau ou article absorbant selon l'une quelconque des revendications 1 à 21, comprenant essentiellement la première partie, la deuxième partie placée sur toute une face principale de la première partie, une couche support adhérant à l'autre face principale de la première partie, avec des orifices perçant la deuxième partie et toute l'épaisseur de la première partie jusqu'à, mais sans inclure, la couche support, et une couche d'hydrogel continue additionnelle placée entre la première partie et la couche support, les orifices perçant également la couche d'hydrogel continue additionnelle.

26. Matériau ou article absorbant selon l'une quelconque des revendications 1 à 21, comprenant essentiellement la première partie, la deuxième partie placée dans des enfoncements présents dans une face principale de la première partie de sorte que la deuxième partie recouvre une partie mais pas toute ladite face principale de la première partie et une couche support adhérant à l'autre face principale de la première partie.

27. Matériau ou article absorbant selon l'une quelconque des revendications 1 à 21, comprenant essentiellement la première partie, la deuxième partie placée dans des enfoncements présents dans une face principale de la première partie de sorte que la deuxième partie recouvre une partie mais pas toute ladite face principale de la première partie, une couche support adhérant à l'autre face principale de la première partie, et une couche d'hydrogel continue additionnelle placée entre la première partie et la couche support.

28. Matériau ou article absorbant selon l'une quelconque des revendications 1 à 21, comprenant essentiellement la première partie, la deuxième partie imprimée sur une partie mais pas toute une face principale de la première partie et une couche support adhérant à l'autre face principale de la première partie.

29. Matériau ou article absorbant selon l'une quelconque des revendications 1 à 21, comprenant essentiellement la première partie, la deuxième partie imprimée sur une partie mais pas toute une face principale de la première partie, une couche support adhérant à l'autre face principale de la première partie, et une couche d'hydrogel continue additionnelle placée entre la première partie et la couche support.

30. Procédé de préparation d'une feuille composite d'hydrogel absorbante, comprenant l'association d'une première partie couche absorbante hydrophile ayant deux faces principales et ayant une structure cellulaire interne et une deuxième partie comprenant une matrice polymère hydrophile plastifiée souple ayant une structure interne qui est relativement continue par rapport à la structure interne de la première partie, à condition qu'au moins une de ladite première partie et de ladite deuxième partie comprenne un hydrogel de sorte que la deuxième partie :
(a) s'infiltre dans certains mais tous les vides cellulaires de la première partie couche absorbante hydrophile sur une première face principale de la partie couche absorbante ; et/ou
(b) recouvre une face principale de la partie couche absorbante, où une partie mais pas toute la première partie est en fait exposée pour permettre l'absorption d'eau externe ou d'un autre liquide dans les vides cellulaires par la face de la feuille correspondant à ladite face principale de la couche absorbante, et où la première partie et la deuxième partie ne sont pas intégralement formées ensemble en une étape à partir d'un mélange unique de pré-gel.

31. Procédé selon la revendication 30, comprenant en outre l'association de la première partie et de la deuxième partie de la feuille composite d'hydrogel à au moins une autre couche ou composant avant, pendant ou après l'association de la première partie et de la deuxième partie de la feuille composite d'hydrogel.

32. Procédé selon la revendication 31, dans lequel l'au moins un(e) autre couche ou composant comprend une couche support.

33. Procédé selon l'une quelconque des revendications 30 à 32, dans lequel l'association des parties est obtenue par :
(a) la formation de la première partie du composite sous forme de couche de mousse à l'état durci solide ;
(b) l'application sur une de ces surfaces d'un précurseur liquide de pré-gel pour la deuxième partie du composite ;
(c) l'infiltration du précurseur liquide de pré-gel dans les pores de la première partie selon l'objectif souhaité ; et
(d) le durcissement ultérieur de la deuxième partie pour donner la feuille composite.

34. Procédé selon l'une quelconque des revendications 30 à 32, dans lequel l'association des parties est obtenue par l'adhérence ou la stratification de manière classique de zones préformées de la deuxième partie du composite à une partie couche préformée du composite.

35. Procédé selon l'une quelconque des revendications 30 à 34, dans lequel la feuille composite d'hydrogel absorbante est utilisée dans le matériau ou article absorbant selon l'une quelconque des revendications précédentes.

36. Matériau ou article absorbant comprenant une feuille composite d'hydrogel absorbante préparée par le procédé selon l'une quelconque des revendications 30 à 34.

37. Electrode biomédicale comprenant un élément de distribution de courant électriquement conducteur adapté pour un raccordement électrique à un appareil électrique, et une portion pouvant être en contact avec la peau électriquement conductrice associée à l'élément de distribution de courant, où le courant électrique peut passer entre l'appareil électrique et la peau d'un porteur lorsque l'électrode est en marche, où la portion pouvant être en contact avec la peau électriquement conductrice comprend un matériau ou article absorbant selon l'une quelconque des revendications 1 à 29 ou 36.

38. Pansement pour plaie ou brûlure comprenant un élément absorbant adapté au contact de la peau d'un porteur à l'endroit d'une plaie ou d'une brûlure, et un élément de support de feuille supportant l'élément absorbant, l'élément support de feuille comprenant une partie qui s'étend au-delà de l'élément absorbant et définie une surface dirigée vers la peau qui porte un adhésif sensible à la pression pour sécuriser le pansement sur la peau d'un porteur, où l'élément absorbant comprend un composite d'hydrogel absorbant ou une combinaison d'hydrogel/couche de libération selon l'une quelconque des revendications 1 à 29 ou 36.
